# EUROPEAN PATENT APPLICATION

(11) **EP 1 935 984 A1**
(43) Date of publication of application: **25.06.2008**
(21) Application number: 06126658.1
(22) Date of filing: 20.12.2006
(51) Int. Cl.: C12Q 1/18, C12Q 1/25

(54) **Screening method for the identification of a preservative**

(71) Applicant: UNILEVER N.V., 3013 AL Rotterdam (NL)
(72) Inventor: ter Beek, Alex, 1012 WX Amsterdam (NL); Brul, Stanley, 3133 AT Vlaardingen (NL); van der Vaart, Jan Marcel, 3133 AT Vlaardingen (NL)
(74) Representative: Tjon, Hon Kong Guno

(57) **Abstract**

The invention relates to a screening method for the identification of a preservative compound by contacting a potential preservative compound, preferably in the presence of a weak acid with a biological target,said biological target comprising a specific polypeptide and identifying a preservative compound when an effect of the potential preservative compound on the biological target is identified.

## Description

### Field of the invention

The invention relates to a screening method for the identification of a preservative compound by contacting a potential preservative compound, preferably in the presence of a weak acid with a biological target, said biological target comprising a specific polypeptide and identifying a preservative compound when an effect of the potential preservative compound on the biological target is identified.

### Background of the invention

The main goal of the food industry is to provide the consumers with high quality and safe food products. To establish safety, the food industry uses food preservatives and/or preservation techniques to prevent the growth of unwanted microorganisms. However, high concentrations of preservatives are harmful to food quality and too low concentrations might promote the development of resistant species. Therefore, the food industry constantly needs to develop more efficient and different antimicrobials.

Weak organic acids (like sorbic-, and acetic acid) are a class of often-used preservatives. They inhibit the growth of many microorganisms, but their exact mode of action is unknown. Understanding the mode of action of weak organic acids may lead to the identification of novel efficient screening methods for the identification of novel preservatives that could be used alone or in combination with a weak acid in order to reduce weak acid resistency and/or to reduce the amount of weak acid needed. Usually screening methods for the identification of novel preservatives include test for growth inhibition using low numbers of microorganism cells in the presence of several concentrations of a potential preservative compounds. Such tests are typically carried out in microtiter plates. These tests are generally non-specific and therefore not efficient (Brehm-Stecher and Johnson, (2004), Single cell microbiology: tools, technologies, and applications. Microbiol. Mol. Biol. Rev. 68:538-559).

Therefore there is still a need for identifying novel and more efficient screening methods.

### Description of the invention

### Screening method

In a first aspect, the invention provides a screening method for the identification of a preservative compound, which method comprises:
(a) contacting a potential preservative compound, preferably in the presence of a weak acid with a biological target, said biological target comprising a polypeptide selected from the group consisting of:
   (i) YhcA having an amino acid sequence which has at least about 16% identity with the amino acid sequence of SEQ ID NO:1,
   (ii) YmfM having an amino acid sequence which has at least about 28% identity with the amino acid sequence of SEQ ID NO:2 and,
   (iii) a phenolic acid decarboxylase PadC having an amino acid sequence which has at least about 45% identity with the amino acid sequence of SEQ ID NO:3,
   and subsequently,
(b) identifying an effect such as reduction of a polypeptide activity which the potential preservative compound has on the biological target; whereby the effect is indicative for preservative activity of the potential preservative compound.

Two complementing strategies (see examples) led to the identification of three polypeptides as defined above under (i), (ii) and (iii) as potentially involved in weak acid resistance. The invention uses this result to develop a screening assay wherein at least one of these three polypeptides is used as sensor to identify new powerful preservative compounds and/or preservatives that may be used in combination with a weak acid.

A preservative is a natural or chemical compound commonly added in products such as food product and that kills or inhibits the growth of microorganisms present in food. Any potential preservative compound may be contacted with a biological target in the method of the invention. The potential preservative compound may be any compound, which is suspected to prevent or inhibit the proliferation of microbial cells such as bacterial or fungal cells. In particular potential preservative compounds, which are suspected to prevent or inhibit the proliferation of bacterial cells are preferred. In a preferred embodiment, the potential preservative compound is a compound which when used in combination with a weak acid will lead to a lowering of the amount of weak acid needed in order to obtain the same effect on the targeted microbial cells (synergistic combination). More preferably, the lowering is of at least 5%, 10%, 15%, 20%, 25% , 30%, 35%, 40%, 45%, 50%, 60%, 70%, 80%, 90%, 95%, 98%, 99% or more of the concentration of weak acid needed. To isolate new potential preservative compounds using the screening method of the invention, microbial, fungal or natural extracts may be used. Alternatively, chemical libraries may be used.

In a preferred embodiment, the potential preservative compound is contacted with the biological target in the presence of a weak acid. In the context of this invention, weak acid means at least one acid selected from the group consisting of: sorbic acid, benzoic acid, acetic acid, cinnamic acid, citric acid, lactic acid. Preferably, the weak acid is sorbic acid.

### Polypeptides present in the biological target used in the screening method of the invention and being the object of the invention as such

The polypeptides present in the biological target used in the screening method and being the object of the invention as such are selected from the list consisting of:
(i) YhcA having an amino acid sequence which has at least about 16% identity with the amino acid sequence of SEQ ID NO:1,
(ii) YmfM having an amino acid sequence which has at least about 28% identity with the amino acid sequence of SEQ ID NO:2 and,
(iii) a phenolic acid decarboxylase PadC having an amino acid sequence which has at least about 45% identity with the amino acid sequence of SEQ ID NO:3.

Each of these polypeptides is further defined below.

### YhcA

The gene encoding the polypeptide YhcA was identified by arrays (see example) as being induced upon addition of sorbic acid. The YhcA polypeptide is a possible multi-drug efflux pump and comprises thirteen putative transmembrane domains. By analysis using several databases such as NCBI and BLAST via www.microbesonline.org, YhcA was identified as both a member of the drug resistance transporter EmrB/QacA subfamily and the major facilitator superfamily. The functionality of this polypeptide was confirmed in a null mutant (strain ATB004) which was found sensitive for sorbic acid.

Accordingly, the YhcA polypeptide present in the biological target used in the screening method of the invention has an amino acid sequence which has at least 16% identity with the amino acid sequence of SEQ ID NO: 1. This polypeptide as such is a further aspect of the invention. The amino acid sequence of YhcA from *Bacillus subtilis* is given in SEQ ID NO:1. The cDNA (or nucleic acid) sequence encoding the amino acid sequence of SEQ ID NO: 1 is given in SEQ ID NO:4.

The activity of the YhcA polypeptide is preferably assessed using any one of the following assays:
first the isolated YhcA is inactivated in the microorganism it derives from. If the obtained mutant shows (increased) sensitivity for a weak acid, preferably sorbic acid, the isolated YchA is said functional and useful for the screening method of the invention. Increased sensitivity for a weak acid means that the growth of the mutant will be at least 10% more inhibited than the growth of the microorganism it originates from using at least 0.05 mM undissociated weak acid. Preferably, the weak acid is sorbic acid in this assay.

Second, the isolated YchA is overexpressed in the microorganism it derives from (see section entitled host cell). Alternatively the isolated YchA is overexpressed in a *Bacillus subtilis* strain. If the obtained microorganism shows an increase in the resistance for a weak acid, preferably sorbic acid, the isolated YchA is said funtional and useful for the screening method of the invention. An increase in the resistance for a weak acid means that the growth of the obtained microorganism will not be affected by at least 0.05 mM undissociated weak acid. Preferably, the weak acid is sorbic acid in this assay.

Third, the microorganism (over)expressing the YchA isolated or the mutant wherein the YchA has been inactived are incubated for approximatively one hour with a fluorescent probe, optionally in the presence of a weak acid, preferably sorbic acid.

Preferred probes are fluoresceindiacetate and its derivates. Such probes are comercially available via Molecular Probes. After washing the microorganism, the measurement of the fluorescence outside of the microorganism (as an indication of the percentage of the fluorescent probe secreted outside of the cell) gives an indication of the functionality of the YchA. The measurement is carried out for a mutant, a microorganism (over)expressing the YchA and optionally on control cells not expressing YhcA at the beginning and end of the incubation. If the mutant (having inactivated YchA) is impaired and/or the microorganisms (over)expressing the endogenous YchA have (highly) enhanced secretion capacities of the fluorescent probe, it will indicate that the YchA is functional and useful for the screening method of the invention. Impaired means approximatively less than 10% of the original amount of fluorescent probe is found outside the cell. Enhanced secretion capacity means approximatively at least 30% of the original amount of fluorescent probe is found outside the cell. Highly enhanced secretion capacity means approximatively at least 50% of the original amount of fluorescent probe is found outside the cell. This assay is preferably carried out in *Bacillus,* more preferably in *Bacillus subtilis.*

These three assays are named YhcA assays later on. When the YhcA is found functional in at least one of these YhcA assays, it may be used in the screening method of the invention.

According to an even more preferred embodiment, the YhcA polypeptide has at least 18%, 20%, 25%, 30%, 35%, 40%, 41%, 43%, 45% or 50%, even more preferably at least 55%, 60%, 65%, 70% , 75%, 80%, 85%, 90%, 95%, 97%, 98%, or at least 99% identity with the amino acid sequence of SEQ ID NO: 1.

In another preferred embodiment, the YhcA polypeptide comprises a fragment, said fragment having at least 16% identiteit with fragment 1, fragment 1 consisting of amino acid number 2 till 178 of SEQ ID NO:1. More preferably, the YhcA polypeptide comprises a fragment, said fragment having at least at least 20%, 25%, 30%, 40%, 50%, 60%, 70%, 80%, 90% or at least 98% identiteit with fragment 1 as defined above.

In one preferred embodiment, the polypeptide of the invention and present in the biological target used in the screening method of the invention comprises an amino acid sequence which is 100% identical to the amino acid sequence of SEQ ID NO:1.

Most preferably, the polypeptide has the amino acid sequence of SEQ ID NO:1. Accordingly, the polypeptide comprising or having the amino acid sequence of SEQ ID NO: as such is also a preferred polypeptide of the invention.

### YmfM

The gene encoding the polypeptide YmfM was identified by transposition mutagenesis of a bank (see example) as conferring resistance to sorbic acid. The function of the YmfM polypeptide is not known. However, the BLAST analysis shows it has similarity with other transcriptional regulators. The functionality of this polypeptide was confirmed in a null mutant (ATB011) which was found sensitive for sorbic acid. Accordingly, the YmfM polypeptide present in the biological target used in the screening method of the invention has an amino acid sequence which has at least 28% identity with the amino acid sequence of SEQ ID NO:2. This polypeptide as such is a further aspect of the invention. The amino acid sequence of YmfM from *Bacillus subtilis* is given in SEQ ID NO:2. The cDNA (or nucleic acid) sequence encoding the amino acid sequence of SEQ ID NO:2 is given in SEQ ID NO:5.

The activity of the YmfM polypeptide is preferably assessed using any of the following assays which are similar with the first and second YhcA assays as earlier described (inactivation and overexpression and test of effect of weak acid).

In addition as a third assay, if the phenotype of the mutant (inactivated YmfM) when exposed to stress conditions has a round shape and/or lyse easily, it may give a first indication as to the functionality of the YmfM. Stress conditions are preferably glucose depletion or sorbate stress (at least 3mM at pH 6.4 or at least 0.05mM undissociated sorbic acid).

These assays are further referred to as YmfM assays. When the YmfM is found functional in at least one of these YmfM assays, it may be used in the screening method of the invention.

According to an even more preferred embodiment, the YmfM polypeptide has at least 30%, 35%, 40%, 45%, 50%, 51%, 52%, 55%, 60%, 65%, 70%, 75%, 80%, 85%, 90%, 95% or 97%, even more preferably at least 98%, 99% identity with the amino acid sequence of SEQ ID NO:2.

In one preferred embodiment, the polypeptide of the invention and present in the biological target used in the screening method of the invention comprises an amino acid sequence which is 100% identical to the amino acid sequence of SEQ ID NO:2. Most preferably, the polypeptide has the amino acid sequence of SEQ ID NO:2. Accordingly, the polypeptide comprising or having the amino acid sequence of SEQ ID NO:2 as such is also a preferred polypeptide of the invention.

In another preferred embodiment, the YmfM polypeptide comprises a fragment, said fragment having at least 21 % identiteit with fragment 2, fragment 2 consisting of amino acid number 1 till 150 of SEQ ID NO:2. More preferably, the YmfM polypeptide comprises a fragment, said fragment having at least at least 25%, 30%, 40%, 50%, 60%, 70%, 80%, 90% or at least 98% identiteit with fragment 2 as defined above.

### PadC

The gene encoding the polypeptide PadC was identified using arrays (see example) as being induced upon addition of sorbic acid. The PadC polypeptide is a phenolic acid decarboxylase. No null mutant could be obtained. The mutant is either not viable or the function of the PadC polypeptide is essential. Accordingly, the PadC polypeptide present in the biological target used in the screening method of the invention has an amino acid sequence which has at least 45% identity with the amino acid sequence of SEQ ID NO:3. This polypeptide as such is a further aspect of the invention. The amino acid sequence of PadC from *Bacillus subtilis* is given in SEQ ID NO:3. The cDNA (or nucleic acid) sequence encoding the amino acid sequence of SEQ ID NO:3 is given in SEQ ID NO:6.

The activity of the PadC polypeptide is preferably assessed using any one of the following assays:
an *in vivo* assay wherein a known substrate of PadC is added to the medium wherein a PadC expressing microorganism (for example *Bacillus subtilis*) is cultivated. Alternatively, the substrate is added to the isolated PadC polypeptide (in vitro assay). Known substrate of PadC include organic acids such as phenolic acids. Detectable amounts of the product formed by PadC using the given susbtrate will indicate that the PadC is functional and may be used in the screening method of the invention. The formed product may be detected using gas chromatography and/or HPLC techniques. This assay is referred to as the *in vivo* or *in vitro* PadC assay later on.

As a second assay, one may use an assay which is similar to the second YhcA or YmfM assay: overexpression of PadC in a microorganism already expressing PadC (*Bacillus subtilis,* for example). Alternatively to overexpress PadC, any repressor of PadC may be inactivated, or any activator of PadC may be overexpressed. As repressor of PadC, one may use *padR*. PadR codes for the repressor of the padC gene (confere Bacillus subtilis database: http://genolist.pasteur.fr/SubtiList/, characterization of PadR provided by: J.-F. Cavin, V. Dartois, C.Divies). A padR mutant strain may be used as strain overexpressing PadC.

Subsequently, as in the former PadC assay, a known substrate is added. An increased formation of the product of at least 10% as compared to the amount of product detected in the microorganism not overexpressing PadC will indicate that the PadC is functional and may be used in the screening process of the invention.

These assays are further referred to as PadC assays. When the PadC is found functional in at least one of these PadC assays, it may be used in the screening method of the invention.

According to an even more preferred embodiment, the PadC polypeptide has at least 50%, 55%, 60%, 65%, 70%, 75%, 80% or 85%, even more preferably at least 90%, 92%, 95%, 97% , 98%, 98.5%, 99%, 99.5% or 99.9% identity with the amino acid sequence of SEQ ID NO:3.

In one preferred embodiment, the polypeptide of the invention and present in the biological target used in the screening method of the invention comprises an amino acid sequence which is 100% identical to the amino acid sequence of SEQ ID NO:3. Most preferably, the polypeptide has the amino acid sequence of SEQ ID NO:3. Accordingly, the polypeptide comprising or having the amino acid sequence of SEQ ID NO:3 as such is also a preferred polypeptide of the invention.

In the context of the invention, percentage of identity between polypeptides was calculated using version 2.2.15 of the BLAST program using standard settings (http://www.ncbi.nlm.nih.gov/BLAST/) except for: No. of Descriptions:10000, No. of Alignments: 10000 in the format box.

The skilled person will understand that the screening method of the invention could be applied using a biological target comprising at least one of the polypeptides as identified above. Each of these polypeptides may be obtained from other microbial organisms, eg, from other prokaryotes than *Bacillus subtilis.* Preferred prokaryotes are bacteria. More preferred bacteria are Gram positive bacteria. Even more preferred Gram positive bacteria are *Bacillus* species. Even more preferably, the *Bacillus* species is a *Bacillus subtilis* species. Even more preferably, the *Bacillus subtilis* is a *Bacillus subtilis* 168 strain, even more preferably the PB2 strain. The sequence of this strain is available via http://genolist.pasteur.fr/Subtilist. This strain may also be obtained via Bacillus Genetic stock center.

Other preferred prokaryotes are for YhcA: *Bacillus licheniformis, Bacillus cereus, Bacillus thuringiensis, Bacillus anthracis, Bacillus clausii, Bacillus halodurans, Bacillus amylliquefaciens, Bacillus firmus, Staphylococcus, Listeria, Lactobacilli, Pediococcus, Enterococcus, Streptococcus, Lactococcus, Clostridia, Rhodococcus, Mycobacterium, Streptomyces, Bifidobacteria, Pseudomonas, Xanthomonas, Salmonella, E. coli.*

Other preferred prokaryotes are for YmfM: *Bacillus licheniformis, Bacillus cereus, Bacillus thuringiensis, Lactobacilli, Moorella thermoacetica, Roseflexus, Oenococcus oeni.*

Other preferred prokaryotes are for PadC: *Bacillus licheniformis, Bacillus pumilus, Lactobacilli, Erwinia, Bifidobacteria, Pseudomonas.*

Any host cell expressing any of these three polypeptides of the invention is a potential target of the potential preservative screened in the method of the invention. Such polypeptides may be obtained using state of the art molecular biology techniques. More preferably, the polypeptide used is obtained from a *Bacillus subtilis* strain and most preferably from the PB2 strain. It is also encompassed by the invention to isolate several YhcA, and/or YmfM and/or PadC polypeptides from one single organism. Accordingly, all these polypeptides are also as such part of the invention.

According to another preferred embodiment, the polypeptide of the invention, which is also preferably used in the screening method of the invention is a variant of any one of the polypeptide sequences of YhcA, YmfM, and/or PadC as defined before. A variant polypeptide may be a non-naturally occurring form of the polypeptide. A polypeptide variant may differ in some engineered way from the polypeptide isolated from its native source. A variant may be made by site-directed mutagenesis starting from the amino acid sequence of SEQ ID NO:1, SEQ ID NO: 2 and/or SEQ ID NO:3 or from the nucleic acid sequence encoding the amino acid sequence of SEQ ID NO:1, SEQ ID NO:2 and/or SEQ ID NO:3 which are respectively SEQ ID NO:4, SEQ ID NO:5 and/or SEQ ID NO:6. Preferably, the polypeptide variant contains mutations that do not alter the biological function of the encoded polypeptide. According to a preferred embodiment, the polypeptide variant has an enhanced polypeptide activity. A polypeptide variant with an enhanced polypeptide activity, is a polypeptide exhibiting a polypeptide activity, which is increased compared to the polypeptide activity of its wild type counterpart measured in a given assay. Preferably, the assay is any one of those described earlier herein for assessing polypeptide activity and named YhcA assay, YmfM assay and PadC *in vivo* and/or *in vitro* assays. According to a more preferred embodiment, the polypeptide YhcA, YmfM and/or PadC variant has an enhanced activity compared to the polypeptide having SEQ ID NO: 1, SEQ ID NO:2 and/or SEQ ID NO:3 respectively as measured in the YhcA, YmfM and *in vivo* and/or *in vitro* PadC assays as defined above. According to an even more preferred embodiment, the polypeptide variant has an enhanced activity compared to the activity of *Bacillus subtilis* corresponding polypeptides derivatives as preferably measured using any of the YchA, YmfM and *in vivo* and/or *in vitro* PadC assays as earlier defined. Polypeptides with enhanced activities are very useful since they can be advantageously used in the screening assay of the invention. It is expected that the screening assay would be more sensitive when this kind of variant polypeptide is being used.

In the context of the invention, a biological target may be any suited biological system. Depending on the biological target, the potential preservative compound to be tested, the skilled person will try several duration of the contacting step, several amounts of potential preservative compounds and/or several amounts of the biological target and preferably several amounts of weak acid. In a preferred embodiment, the contacting step is carried out in a simple container, such as a microtitre plate. More preferably in these assays, the weak acid is sorbic acid.

In a first preferred embodiment, the biological target is a cell. In this embodiment, the contacting step of the method of the invention is carried out *in vivo* (*in vivo* screening method). The cell can be any cell as long as it expresses at least one of the three polypeptides as defined above under (i), (ii) or (iii). The cell may natively express at least one of the genes encoding these polypeptides (endogenous expression). In this embodiment, the cell is preferably a *Bacillus* cell. More preferably, a *Bacillus subtilis* cell. Alternatively, at least one of the genes encoding these polypeptides may be brought into the cell via recombinant DNA technology as exemplified in the section entitled host cell (non endogenous expression, heterologous expression). It is also possible that the cell natively expresses at least one of the genes encoding these polypeptides and that overexpression of at least one of these genes is achieved by recombinant DNA technology (see section host cell).

This type of assay is typically performed in a microtiter plate as a long term or as a short term assay. A long term assay may have a duration of approximatively 5 days, starting with a cell number of approximatively 10 cells / well, 0.005 mM undissociated weak acid and 0.005mM of a potential preservative compound. A short term assay may have a duration of at least 4 hours, starting with a cell number of approximatively 4x10⁶ cells /well, 0.05 mM undissociated weak acid and 0.010mM of a potential preservative compound.

Alternatively, and according to a second preferred embodiment, the biological target is a composition comprising the polypeptide as defined under (iii) and the contacting step is carried out *in vitro (in vitro* screening method).

In the second step of the screening method of the invention, an effect is identified such as reduction of a polypeptide activity which the potential preservative compound has on the biological target, preferably in the presence of a weak acid. This effect is indicative for preservative activity of the potential preservative compound. When a weak acid is present, this effect is indicative for a synergistic preservative effect between the weak acid and the potential preservative compound, preferably a synergy between sorbic acid and the potential preservative compound. In a preferred embodiment, the effect is a polypeptide activity.

Polypeptide activity may be any detectable and measurable activities of a polypeptide, e.g. enzyme activity, inhibitory activity, biosynthetic activity, transporter activity, cell division activity, transcriptional activity or translational activity. According to a preferred embodiment, the activity of the polypeptide means enzymatic activity. More preferably, polypeptide activity means decarboxylase or PadC activity.

Depending on the identity of the polypeptide used in the screening method of the invention, the skilled person would know which assay is the best suited to assess the activity of the chosen polypeptide. The effect of the potential preservative compound may be determined using an assay, preferably a microtitre plate assay, wherein the activity of the polypeptide is determined with any simple assay known to the skilled person, e.g. an assay based on radioactivity, fluorescence or by HPLC.

When polypeptide activity means enzymatic activity, enzymatic activity is preferably assessed using an assay detecting decarboxylase or PadC activity, especially if the polypeptide used is a PadC enzyme. A preferred assay to be used for the detection of decarboxylase activity is either the *in vivo* or *in vitro* PadC assay as earlier defined. According to this preferred embodiment, the biological target comprises a PadC polypeptide as earlier defined herein and the assay is *the in vitro* PadC assay.

The reduction of polypeptide activity is preferably assessed by testing the polypeptide activity in the presence and in the absence of the potential preservative compound and preferably in the presence of a weak acid. More preferably, the weak acid is sorbic acid.

Preferably, the activity of the polypeptide in the presence of the potential preservative compound is reduced compared to the activity of the polypeptide in the absence of the potential preservative compound. More preferably, this polypeptide activity is measured (presence or absence of the potential preservative compound) in the presence of a weak acid, most preferably sorbic acid. The polypeptide activity may be reduced completely, i.e. 100%, or in part. For instance, it may be reduced for more than 10%, 20%, 30%, 40%, 50%, 60% or 70%, or for more than 75%, 80%, 85% or 90% or for more than 92%, 94%, 96%, 98%, or 99%. In this paragraph, the polypeptide activity tested is preferably PadC, more preferably using *the in vitro* PadC assay.

According to a preferred embodiment, a reduction of the activity of the polypeptide in the presence of the potential preservative compound of at least 10% compared to the activity of the polypeptide in the absence of said potential preservative compound is indicative for preservative activity of the potential preservative compound. In this paragraph, the polypeptide activity tested is preferably PadC, more preferably using *the in vitro* PadC assay. Even more preferably, this polypeptide activity is measured (presence or absence of the potential preservative compound) in the presence of a weak acid, most preferably sorbic acid. When a weak acid is present, a reduction of the activity of the polypeptide in the presence of the potential preservative compound of at least 10% compared to the activity of the polypeptide in the absence of said potential preservative compound is indicative for synergistic preservative activity of the potential preservative compound together with the weak acid.

According to a more preferred embodiment, a reduction of the activity of the polypeptide in the presence of the potential preservative compound of at least 20%, even more preferably of at least 30% and most preferably of at least 50% compared to the activity of the polypeptide in the absence of said potential preservative compound is indicative for preservative activity of the potential preservative compound. In this paragraph, the polypeptide activity tested is preferably PadC, more preferably using *the in vitro* PadC assay. Even more preferably, this polypeptide activity is measured (presence or absence of the potential preservative compound) in the presence of a weak acid, most preferably sorbic acid.

Alternatively according to another preferred embodiment, the biological target is a cell as earlier defined herein (see also section entitled host cell) and the effect is such as reduction of a polypeptide activity. When the biological target is a cell instead of a composition comprising a polypeptide, the skilled person will understand that there are many ways of assessing how the potential preservative compound may affect cell behaviour and/or polypeptide activity. Affecting or reducing polypeptide activity has already been earlier defined and is preferably assessed using any of the YhcA, YmfM and/or PadC *in vivo* assays as earlier defined depending of the identity of the polypeptide expressed by the cell. In another preferred embodiment, the polypeptide activity is reflected by the cell behaviour. More preferably, cell behaviour is reflected by the intracellulair pH, and/or cell growth and/or cell death.

The growth ability of a cell may be measured by assessing the optical density of a culture comprising the cell and/or by visualising its morphological aspect microscopically. Microbial growth may be readily monitored by measuring the optical density in a small container (microtiter plate well) using a specific wavelenghth, preferably between 560 and 620 nm. Microscopical observation reveals morphological abnormalities and defects in growth as well as possible lysis or death of the microbe.

According to a preferred embodiment, a reduction of the cell growth in the presence of the potential preservative compound of at least 10% compared to the growth of a similar cell under similar conditions in the absence of the potential preservative compound is indicative for preservative activity of the potential preservative compound. More preferably, the reduction of the cell growth in the presence of the potential preservative compound is of at least 20%, 30%, 40%, 50%, 60%, 80%, 100%. Alternatively or in combination with earlier embodiments relating to cell growth, the growth of the cell with or without the potential preservative compound is evaluated in the presence of a weak acid, even more preferably sorbic acid. This type of growth assay is typically performed in a microtiter plate as a long term or as a short term assay. A long term assay may have a duration of approximatively 5 days, starting with a cell number of approximatively 10 cells / well, 0.005 mM undissociated weak acid and 0.005mM of a potential preservative compound. A short term assay may have a duration of at least 4 hours, starting with a cell number of approximatively 4x10⁶ cells /well, 0.05 mM undissociated weak acid and 0.010mM of a potential preservative compound Several amounts of potential preservative compounds, several amounts of weak acid, several cell amounts and/or several durations are preferably tested.

Alternatively or in combination with former preferred embodiment (cell growth as effect), in a further preferred embodiment, an increase of the cell death in the presence of the potential preservative compound resulting in at least 10% less cells compared to the amount of similar cell under similar conditions in the absence of the potential preservative compound is indicative for preservative activity of the potential preservative compound. More preferably, a reduction of the cell number in the presence of the potential preservative compound results in at least 20%, 30%, 40%, 50%, 60%, 80%, 100% less cells.

Alternatively or in combination with earlier embodiments relating to cell death, the growth of the cell with or without the potential preservative compound is evaluated in the presence of a weak acid, even more preferably sorbic acid. This type of death assay or negative growth assay is typically performed in a microtiter plate as a long term or as a short term assay. A long term assay may have a duration of approximatively 5 days, starting with a cell number of approximatively 10 cells / well, 0.005 mM undissociated weak acid and 0.005mM of a potential preservative compound. A short term assay may have a duration of at least 4 hours, starting with a cell number of approximatively 4x10⁶cells /well, 0.05 mM undissociated weak acid and 0.010mM of a potential preservative compound. Several amounts of potential preservative compounds, several amounts of weak acid, several cell amounts and/or several durations are preferably tested.

Alternatively or in combination with at least one of the last two former embodiments (cell growth, cell death as effects), in a yet further preferred embodiment, a reduction of the intracellulair pH in the presence of the potential preservative compound of at least 0.1 unit compared to the intracellulair pH in the absence of said potential preservative compound under similar conditions is indicative for preservative activity of the potential preservative compound. More preferably, a reduction of at least 0.15, a reduction of at least 0.2, of at least 0.25, of at least 0.3, of at least 0.35, of at least 0.4, of at least 0.5, even more preferably of at least 0.7, even more preferably of at least 1.0, even more preferably of at least 1.3, even more preferably of at least 1.5, even more preferably of at least 1.8, even more preferably of at least 2, even more preferably of at least 2.3, even more preferably of at least 2.5, and most preferably of at least 3 units intracellulair pH.

Intracellulair pH (pHi) may be assessed by several ways. The weak acid distribution added in the screening method is preferably assessed by a ratiometric pHi measurement using a physiological pH indicator, more preferably a pH-sensitive GFP (green fluorescent protein), even more preferably Phfluorin. A Phfluorin is a PH-sensitive GFP from *Aequorea victoria* which has already been described in Miesenbock G et al (Miesenbock G et al, (1998), Nature, 394: 192-195, Genebank accession number AF058694). PH-sensitive GFP was shown to be a specific intracellular pH indicator. The Intracellulair pH ranged between 5 and 8.5 could be monitored using PH-sensitive GFP. The amino acid sequence respectively the nucleic acid sequence of a preferred pH-sensitive mutant are given as SEQ ID NO:9, respectively SEQ ID NO: 10. In order to measure the intracellular pH, cells used as the biological target in the screening method of the invention are first transfected with suitable constructs comprising a nucleotide sequence encoding a PH-sensitive GFP. This type of assay is typically performed in a microtiter plate with 10⁶ to 10⁸ cells, 0.05 mM undissociated weak acid and 0.010mM of a potential preservative compound. A pH-sensitive GFP is expressed in the cytosol of the cell. The fluorescence and the absorbance of the PH-sensitive GFP mutant at several wavelengths is dependent on the surrounding pH and could be visualised by the use of a spectrophotometer (Miesenbock el al and/or Olsen K N, (2002), Applied and Environmental Microbiology, 68: 4145-4147). Several amounts of potential preservative compounds, several amounts of weak acid, several cell amounts and/or several durations are preferably tested. Preferred cells for performing this assay are *Bacillus* cells, more preferably *Bacillus subtilis* cells.

### Nucleic acid sequence

In a further aspect, the invention relates to a nucleic acid sequence coding for a preferred polypeptide as defined in the former section entitled "Preferred polypeptides used in the biological target used in the screening method of the invention and being object of the invention as such" (named hereafter polypeptides of the invention) as:
(i)YhcA having an amino acid sequence which has at least about 16% identity with the amino acid sequence of SEQ ID NO:1,
(ii) YmfM having an amino acid sequence which has at least about 28% identity with the amino acid sequence of SEQ ID NO:2 and/or,
(iii) a phenolic acid decarboxylase PadC having an amino acid sequence which has at least about 45% identity with the amino acid sequence of SEQ ID NO:3. According to another preferred embodiment, the nucleic acid sequence of the invention is a variant of the nucleic acid sequence defined above. Nucleic acid sequence variants may be used for preparing polypeptide variants as defined earlier. A nucleic acid variant may be a fragment of any of the nucleic acid sequences as defined above. A nucleic acid variant may also be a nucleic acid sequence that differs from any of SEQ ID NO:4, SEQ ID NO:5 or SEQ ID NO:6 by virtue of the degeneracy of the genetic code. A nucleic acid variant may also be an allelic variant of SEQ ID NO:4, SEQ ID NO:5 or SEQ ID NO:6. An allelic variant denotes any of two or more alternative forms of a gene occupying the same chromosome locus. A preferred nucleic acid variant is a nucleic acid sequence, which contains silent mutation(s). Alternatively or in combination, a nucleic acid variant may also be obtained by introduction of nucleotide substitutions, which do not give rise to another amino acid sequence of the polypeptide encoded by the nucleic acid sequence, but which corresponds to the codon usage of the host organism intended for production of the polypeptides of the invention. According to a preferred embodiment, the nucleic acid variant encodes a polypeptide still exhibiting its biological function. More preferably, the nucleic acid sequence variant encodes a polypeptide exhibiting a polypeptide activity as assessed in any of the assays defined earlier herein. Even more preferably, the nucleic acid variant encodes a polypeptide with enhanced polypeptide activity as defined earlier. Nucleic acid sequences encoding a polypeptide activity may be isolated from any microorganism.

All these variants can be obtained using techniques known to the skilled person, such as screening of library by hybridisation (southern blotting procedures) under low to medium to high hybridisation conditions with for the nucleic acid sequence SEQ ID NO:4 or SEQ ID NO:5 or SEQ ID NO:6 or a variant thereof which can be used to design a probe. Low to medium to high stringency conditions means prehybridization and hybridization at 42°C in 5X SSPE, 0.3% SDS, 200pg/ml sheared and denatured salmon sperm DNA, and either 25% 35% or 50% formamide for low to medium to high stringencies respectively. Subsequently, the hybridization reaction is washed three times for 30 minutes each using 2XSSC, 0.2%SDS and either 55°C, 65 °C, or 75 °C for low to medium to high stringencies.

In a preferred embodiment, the nucleic acid sequence encoding YhcA has at least about 80% identiteit with the nucleic acid sequence of SEQ ID NO:4. More preferably, the identiteit is at least about 85%, at least about 90%, at least about 95% and at least about 99%.

In a preferred embodiment, the nucleic acid sequence encoding YmfM has at least about 87% identiteit with the nucleic acid sequence of SEQ ID NO:5. More preferably, the identiteit is at least about 89%, at least about 90%, at least about 92%, at least about 94%, at least about 95% at least about 97% and at least about 99%.

In a preferred embodiment, the nucleic acid sequence encoding PadC has at least about 79% identiteit with the nucleic acid sequence of SEQ ID NO:6. More preferably, the identiteit is at least about 80%, at least about 85%, at least about 87%, at least about 90%, at least about 95% at least about 97% and at least about 99%.

The sequence information as provided herein should not be so narrowly construed as to require inclusion of erroneously identified bases. The skilled person is capable of identifying such erroneously identified bases and knows how to correct for such errors.

### Nucleic acid construct, expression vector

In a further aspect, the invention relates to a nucleic acid construct comprising a nucleic acid sequence defined in the former section, said nucleic acid sequence encoding a polypeptide as defined in the section entitled "Preferred polypeptides used in the biological target used in the screening method of the invention and being object of the invention as such" (named hereafter polypeptides of the invention) as:
(i) YhcA having an amino acid sequence which has at least about 16% identity with the amino acid sequence of SEQ ID NO:1,
(ii) YmfM having an amino acid sequence which has at least about 28% identity with the amino acid sequence of SEQ ID NO:2 and/or,
(iii) a phenolic acid decarboxylase PadC having an amino acid sequence which has at least about 45% identity with the amino acid sequence of SEQ ID NO:3.

Optionally, the nucleic acid sequence present in the nucleic acid construct is operably linked to one or more control sequences, which direct the production of the polypeptides of the invention in a suitable expression host.

Operably linked is defined herein as a configuration in which a control sequence is appropriately placed at a position relative to the nucleic acid sequence coding for the polypeptide of the invention such that the control sequence directs the production of the polypeptide of the invention.

Expression will be understood to include any step involved in the production of the polypeptide including, but not limited to transcription, post-transcriptional modification, translation, post-translational modification and secretion.

Nucleic acid construct is defined as a nucleid acid molecule, which is isolated from a naturally occurring gene or which has been modified to contain segments of nucleic acid which are combined or juxtaposed in a manner which would not otherwise exist in nature.

Control sequence is defined herein to include all components, which are necessary or advantageous for the expression of a polypeptide. At a minimum, the control sequences include a promoter and trancriptional and translational stop signals.

The invention also relates to expression vectors comprising the nucleic acid construct of the invention. Preferably, the expression vector comprises the nucleic acid sequence of the invention, which is operably linked to one or more control sequences, which direct the production of the encoded polypeptide in a suitable expression host. At a minimum control sequences include a promoter and transcriptional and translational stop signals. The expression vector may be seen as a recombinant expression vector. The expression vector may be any vector (e.g. plasmic, virus), which can be conveniently subjected to recombinant DNA procedures and can bring about the expression of the nucleic acid sequence encoding the polypeptide. Depending on the identity of the host wherein this expression vector will be introduced and on the origin of the nucleic acid sequence of the invention, the skilled person will know how to choose the most suited expression vector and control sequences.

### Host cell

In a further aspect, the present invention relates to a host cell, which comprises the nucleic acid construct or the expression vector of the invention as defined in the former section. The host cell expresses a polypeptide of the invention as defined in the section entitled "Preferred polypeptides used in the biological target used in the screening method of the invention and being object of the invention as such" (named hereafter polypeptides of the invention) as:
(i) YhcA having an amino acid sequence which has at least about 16% identity with the amino acid sequence of SEQ ID NO:1,
(ii) YmfM having an amino acid sequence which has at least about 28% identity with the amino acid sequence of SEQ ID NO:2 and/or,
(iii) a phenolic acid decarboxylase PadC having an amino acid sequence which has at least about 45% identity with the amino acid sequence of SEQ ID NO:3.

In a more preferred embodiment, the host cell further expresses a pH-sensitive GFP as earlier defined.

The choice of the host cell will to a large extent depend upon the source of the nucleic acid sequence and polypeptide of the invention. Depending on the identity of the host cell, the skilled person would know how to transform it with the construct or vector of the invention.

The host cell may be any microbial, prokaryotic or eukaryotic cell, which is suitable for expression of a polypeptide of the invention. Preferably bacterial, yeast, fungal host cells are used. Each of the microorganism cited as preferred source of any of the polypeptides of the invention used in the screening method of the invention is also a preferred host cell of the invention. More preferably are species from *Bacillus,* even more preferably *Bacillus subtilis.* In an even more preferred embodiment, the Bacillus, preferably Bacillus subtilis cells further expresses a pH-sensitive GFP as earlier defined.

Most of these cells are well-known in the art for overexpressing polypeptides and/or for being a food spoilage microorganism. All cells cited under the section "preferred polypeptides present in the biological target used in the screening method and being the object of the invention as such" are also preferred host cells. The host cell may be considered as a recombinant host cell.

Suitable procedures for transformation of bacterial cells are well known in the art (Sambrook et al, (1989) Molecular cloning a laboratory manual, Cold Spring Harbor). When the cell is *Bacillus subtilis,* a two step transformation method may be advantageously used as described in Kunst F et al (Kunst F et al, (1995), J. Bacteriol., 177: 2403-2407)

Suitable procedures for transformation of filamentous fungus may involve a process comprising protoplast formation, transformation of the protoplast, and regeneration of the cell wall in a manner known to the skilled person. Suitable transformation procedures for *Aspergillus* are described in Yelton et al, 1984, Proceedings of the National Academy of Sciences USA, 81:1470-1474.

According to a preferred embodiment, the host cell hence obtained overexpresses, i.e. produces more than normal amounts of a polypeptide of the invention and/or exhibits a higher polypeptide activity associated with any of these polypeptides than the parental cell this host cell derives from when both cultured and/or assayed under the same conditions.

The polypeptides of the invention and polypeptide activities are as defined in the section entitled "preferred polypeptides present in the biological target used in the screening method and being the object of the invention as such".

"Producing more than normal amount" is herein defined as producing more of a polypeptide of the invention than what the parental host cell the transformed host cell derives from will produce when both types of cells (parental and transformed cells) are cultured under the same conditions. Preferably, the host cell of the invention produces at least 3%, 6%, 10% or 15% more of a polypeptide of the invention than the parental host cell the transformed host cell derives from will produce when both types of cells (parental and transformed cells) are cultured under the same conditions. Also hosts which produce at least 20%, 30%, 40%, 50%, 60%, 70%, 80%, 90%, 100% or 150% more of said polypeptide than the parental cell are preferred. According to another preferred embodiment, the production level of the polypeptide of the host cell of the invention is compared to the production level of the *Bacillus subtilis* PB2 strain, which is taken as control. According to an even more preferred embodiment, when the host cell of the invention is an *Bacillus subtilis* strain, the production level of a polypeptide of the invention of the host cell of the invention is compared to the production level of the PB2 strain, which is taken as control.

The assessment of the production level of a polypeptide of the invention may be performed at the mRNA level by carrying out a Northern Blot or an array analysis and/or at the polypeptide level by carrying out a Western blot. All these methods are well known to the skilled person.

"Exhibiting a higher polypeptide activity" is herein defined as exhibiting a higher polypeptide activity than the one of the parental host cell the transformed host cell derives from using an assay specific for the polypeptide activity. Preferably, the host cell of the invention exhibits at least 3%, 6%, 10% or 15% higher polypeptide activity than the parental host cell the transformed host cell derives from will exhibit as assayed using a specific assay for the polypeptide activity. Also host which exhibits at least 20%, 30%, 40%, 50%, 60%, 70%, 80%, 90%, 100% or 150% more of said activity than the parental cell are preferred. According to another preferred embodiment, the level of polypeptide activity of the host cell of the invention is compared to the corresponding activity of the control strain *Bacillus subtilis* PB2. According to a more preferred embodiment, when the host cell of the invention is a *Bacillus subtillus* strain, the level of polypeptide activity of the host cell of the invention is compared to the corresponding activity of the control strain *Bacillus subtilis* PB2. Preferably, the polypeptide activity is a decarboxylase activity of the PadC polypeptide.

The overexpression may have been achieved by conventional methods known in the art, such as by introducing more copies of a nucleotide sequence of the invention encoding a polypeptide of the invention into the host, be it on a carrier or in the chromosome, than naturally present. Alternatively, the nucleotide sequence encoding the polypeptide of the invention can be overexpressed by fusing it to highly expressed or strong promoter suitable for high level polypeptide expression in the selected organism, or combination of the two approaches. Alternatively or in combination, a polypeptide having an enhanced activity as defined earlier can be overexpressed in the host cell of the invention. This can be done using a strong promoter and/or by introducing multiple copies of the encoding gene into the host. The skilled person will know which strong promoter is the most appropriate depending on the identity of the host cell. Preferably when the host cell is a *Bacillus subtillis* strain, the strong promoter is the IPTG inducible pSpaC promoter (see e.g. Bacillus genetic Stock Centre april 13 2001 new product release. pMUTIN4, A vector useful for Gram-positive Genomics and Chung Y J, (1992) Journal of Bacteriology, 174: 1417-1422).

The overexpressing host cell may be used to produce substantial amounts of a polypeptide of the invention which can subsequently be used in the above described method of the invention:
- in a first preferred embodiment, a polypeptide of the invention is first produced using the host cell of the invention. Accordingly, in a further aspect, the invention relates to a method for producing a polypeptide of the invention as defined above by culturing the host cell of the invention under suitable culture conditions. Subsequently, a biological target comprising the polypeptide produced is contacted in *vitro* with a potential preservative compound as defined earlier herein (referred to as *in vitro* method hereafter). In this embodiment, the polypeptide is preferably PadC.
- in a second preferred embodiment, the contacting step between a polypeptide of the invention and the potential preservative compound is carried out *in vivo* using as biological target the host cell of the invention as defined earlier herein. Therefore, a polypeptide is contacted with a potential preservative compound in step (a) by virtue of its expression in the host cell of the invention.

### Kit

In a further aspect, the invention relates to a kit for carrying out *the in vitro* screening method of the invention as first defined in the description. The kit comprises in separate containers (i) a polypeptide of the invention, preferably a PadC polypeptide and (ii) a substrate for said polypeptide. It may further contain markers and controls. Preferably, the polypeptide present in the kit is any one of the preferred polypeptides defined earlier. Accordingly, in a further aspect, the invention relates to the use of this kit for performing *the in vitro* screening method as first described in the description.

### Preservative compound

In yet another aspect, the invention relates to a preservative compound identified by any method of the invention and to a composition comprising the preservative compound of the invention.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The present invention is further described by the following examples which should not be construed as limiting the scope of the invention.

### Examples

### Example 1: Arrays of sorbic acid treated Bacillus subtilis as a tool to identify genes involved in sorbic acid resistance

### Results

To get a better understanding of the global response of *Bacillus subtilis* enduring sorbic acid stress, DNA microarray analysis was performed. We studied changes in gene expression after 10, 20, 30, 40 and 50 min of exposure to potassium sorbate in comparison to an untreated control. Exponentially growing cells were stressed with 3 mM potassium sorbate, which resulted in a reduction in the growth rate of 26 % (data not shown). Changes in gene expression were studied using spotted oligo-arrays. The microarray consisted of 4100 gene specific 65-mer oligonucleotides, representing 4,100 of the 4,106 possible ORFs in *B. subtilis* (as reported for the *B. subtilis* genome at http://genolist.pasteur.fr/SubtiList/), spotted in duplo onto glass slides. Total RNA, isolated from the sorbate stressed and control cultures of each time-point, was labelled with the fluorescent dyes Cy5-dUTP and Cy3-dUTP respectively and hybridized onto the same slide. Biologically independent experiments were performed in duplo. Following washing, scanning and data pre-processing, significantly up- or downregulated genes were identified. Since the variation in differential expression measurements depends on the intensity (smaller variation at higher- and larger variation at lower fluorescence intensity levels), we applied an intensity-dependent method to identify differentially expressed genes (Yang et al., (2002)a, Genome Biol., 3: 0062). This method uses a sliding window to determine the significantly regulated genes (IZI > 1.96).
Although sorbic acid is not a phenolic acid, we surprisingly found the strong up-regulation of the phenolic acid decarboxylase, *padC* in this cluster (Cavin JF et al., (1998), Appl. Environ Microbiol., 64: 1466-1471). According to the KEGG (Kyoto Encyclopedia of Genes and Genomes, Kaneshisa M, et al (2006), Nucl. Acid Res., 34:354-357) database, this enzyme may also be involved in the degradation and biosynthesis of various metabolites, like arginine, proline, tyrosine, tryptophan and pyrine. We speculate whether *padC* is also able to decarboxylate sorbic acid. We found the gene *yhcA,* encoding a possible multidrug resistance protein in one cluster with highly up-regulated genes. This result was confirmed by real-time RT-PCR (not shown).
Interstingly, we found the gene *yhcA* highly up-regulated in the presence of potassium sorbate. YhcA is a possible multi-drug efflux pump and comprises of thirteen putative trans-membrane domains. YhcA is both a member of the drug resistance transporter EmrB/QacA subfamily and the major facilitator superfamily. A BLASTp sequence search (release 2.2.14) demonstrated that YhcA homologs are found predominantly in prokaryotes, especially *Bacilli.* To investigate the role of this interesting gene in sorbate stress tolerance, we tested the mutant strain ATB004 (*yhcA*) in the presence of different concentrations of potassium sorbate. We followed the growth by measuring the increase in OD₆₀₀ and calculated growth inhibition percentages (Table 1). Strikingly, the *yhcA* mutant strain showed a clearly sensitive phenotype, especially at lower sorbate concentrations. The growth rate of the mutant strain compared to the wild-type was lower, and demonstrates the importance of this gene also in control conditions. When stressed with potassium sorbate the *yhcA* mutant strain did not grow exponentially, but the inhibitory effect of sorbate increased over time, confirming its importance in sorbate stress survival (Table 1).

**Table 1. Susceptibility of a selected mutant strain to sorbic acid stress.**

| Strain | Relevant genotype | Potassium sorbate (mM)^{a} | | | | |
|---|---|---|---|---|---|---|
| | | 0 | 3 | 6 | 15 | 30 |
| | | µ(h⁻¹) | GI% | GI% | GI% | GI % |
| PB2 | - | 0.91 (0.05) | 26.1 (2.5) | 40.7 (2.1) | 63.6 (2.8) | 73.2 (2.0) |
| ATB004^{b} | *yhcA* | 0.68 (0.03) | 45.1 (4.6) | 50.0 (3.9) | 72.0 (4.2) | 79.3 (2.9) |

| | | | | | | |
|---|---|---|---|---|---|---|
| **a.** Potassium sorbate was added to exponentially growing cultures of an OD₆₀₀ of 0.2 at pH 6.4. The optical density of the cultures was followed for 3 hours in a micro titre plate reader. Specific growth rates (µ) were calculated from the increase in optical density between 56 and 108 min after sorbate stress. Growth inhibition (GI) percentages were calculated from the obtained growth rates and are relative to the specific growth rate in the control condition (no addition of potassium sorbate). All conditions were tested in the micro titre plate reader at least in duplo and biologically independent experiments were performed at least twice. The standard deviation is shown in parentheses. **b.** The *yhcA* mutant strain did not grow exponentially in the presence of all tested potassium sorbate concentrations (see also Fig. 2). Calculated growth inhibition percentages are therefore underestimated values. | | | | | | |

### Material and Methods

### Bacterial strains and growth conditions

All *Bacillus subtilis* strains used in this study are derivatives of the laboratory wild-type strain PB2 (kindly provided by C.W. Price) and are listed in Table 4. Mutant strains ATB002 (*ureC*), ATB007 (*sigL*) and ATB008 (*sigM*) were obtained by transformation of strain PB2 with chromosomal DNA of strains SF168U (Cruz-Ramos H., et al., (1997) J. Bacteriol., 179:3371-3373), QB5505 (Debarbouille M., et al., (1991), Proc. Natl. Acad. Sci., 181: 2059-2066) and AM1447 (Thackray PD and Moir A, (2003), J. Bacteriol., 185: 3491-3498), respectively. To obtain mutant strains ATB001 (*fabHB*), ATB003 (*ycsF*), ATB004 (*yhcA*), ATB005 (*yhcB*) and ATB006 (*yxkJ*), the wild-type strain PB2 was transformed with chromosomal DNA of strains YHFBd, YCSFd, YHCAd, YHCBd and YXKJd, respectively, which were all received from the Japanese Consortium for Functional Analysis of the *B. subtilis* Genome (JAFAN, http://bacillus.genome.ad.jp/). Transformants were selected on LB (Luria-Bertani) agar plates containing appropriate antibiotics, after overnight incubation at 37°C. Depending on the strain, the used antibiotics were chloramphenicol (6 µg/ml), erythromycin (0.5 µg/ml), spectinomycin (100 µg/ml) or kanamycin (10 µg/ml). Isolation of chromosomal DNA was performed according to Ward JB. (Ward JB et al, (1973), J. Bacteriol., 116: 719-726) and transformations were carried out as described by Kunst et al (Kunst F. et al, (1995) J. Bacteriol., 177: 2403-2407).
All *B. subtilis* strains were cultivated in a defined minimal medium as described by Hu et al. ((Hu P et al (1999) J. Bacteriol., 181: 5042-5050), as modified from Neidhardt et al.(Neihardt FC et al, (1974) J. Bacteriol., 119: 736-747). The medium contains 1.32 mM K₂HPO₄, 0.4 mM MgCl₂, 0.276 mM K₂SO₄, 0.010 mM FeSO₄, 0.14 mM CaCl₂, 4 mM tricine, 0.1 mM MnCl₂ and 0.2448 mM tryptophane. The defined media is further supplemented with micronutrients, containing 3*10⁻⁶ mM (NH₄)₆Mo₇O₂₄, 4*10⁻⁴ mM H₃BO₃, 3*10⁻⁵ mM CoCl₂, 1*10⁻⁵ mM CuSO₄ and 1*10⁻⁵ mM ZnSO₄. The medium is buffered with 80 mM 3-[N-morpholono]propanesulfonic acid (MOPS) and the pH is set to 5.9, 6.4, 7.4 or 7.8 with KOH. As carbon- and nitrogen-sources 5 mM glucose, 10 mM glutamate and 10 mM NH₄Cl are used. All strains were grown exponentially to an optical density at 600 nm (OD₆₀₀) of 0.2, transferred to a SpectroMax Plus microtitre plate reader (Molecular Devices Corp.) and, depending on the used pH, stressed with various concentrations of potassium sorbate (1.25, 2.5, 3, 5, 6, 7, 10, 15, 20, 30, 40, 80 or 125 mM). Cells were further cultivated in the microtitre plate reader under rigorous shaking at 37 °C for 180 min. All conditions were tested in the microtitre plate reader at least in duplo and biologically independent experiments were performed at least twice. Long-term stress survival of the strains was tested by spotting serial dilutions on defined minimal medium agar plates of pH 6.4 with or without potassium sorbate. Cells were grown exponentially to an OD₆₀₀ of 0.2 and 10-fold serial dilutions were spotted onto plates containing 0, 3, 6, 15 and 30 mM potassium sorbate. After incubation for 24 h at 37 °C, the plates were photographed.

### Preparation of total RNA for transcriptome analysis

An exponentially growing culture of *B. subtilis* wild-type strain PB2 was split in two and inoculated in well-controlled batch-fermentors to an OD₆₀₀ of 0.05. The cultures were grown at 37°C in defined medium at pH 6.4 with an aeration rate of 0.5 litres/min and fast stirring (200 rpm). At an OD₆₀₀ of 0.2, one culture was sub-lethally stressed with 3 mM potassium sorbate. Samples of 20 ml were withdrawn from both the treated and control cultures at 0, 10, 20, 30, 40, and 50 min after addition of potassium sorbate. The cells were collected using a vacuum-filtering set-up, immediately quenched in liquid nitrogen and stored at -80 °C to await RNA extraction. The whole procedure took no longer than 50 s. Two biologically independent experiments were performed. Total RNA was isolated using the FastRNA Pro Blue kit and FastPrep instrument (Qbiogene) following the manufacturer's protocol. After (RNase free) DNase I (Fermentas) treatment, the quantity and integrity of the samples was assessed spectrometricly on a Nanodrop (Isogen Life Science) and with a LabChip kit on a 2100 Bioanalyser (Agilent Technologies).

### DNA microarray construction

The *Bacillus subtilis* DNA microarrays were obtained by spotting a *B. subtilis* oligonucleotide library (Sigma-Genosys) in duplo onto UltraGAPS glass slides (Coming) with a Lucidea Array Spotter (GE Healthcare) according to standard protocols. The library contains 4,100 gene specific 65-mer oligonucleotides, representing 4,100 possible ORFs in *B. subtilis.* The spotted oligonucleotides were immobilized onto the microarray by UV cross-linking.

### Synthesis of labelled cDNA, hybridization and scanning of the DNA microarrays

Superscript II reverse transcriptase (Invotrogen) was used to synthesize labelled cDNA from total RNA samples by direct incorporation of Cy3- or Cy5-labelled dUTP into cDNA. The reaction mixture performed in First-Strand buffer contained 12 µg of total RNA, 0.5 µg of random hexamers (GE Healthcare), 400 Units of Superscript II reverse transcriptase, 10 mM dithiothreitol, 0.5 mM dATP, dCTP, dGTP and 0.2 mM dTTP (GE Healthcare), and 0.07 mM Cy3- or Cy5-dUTP (GE Healthcare). Control and sorbate treated samples were incorporated with Cy3- and Cy5-labelled dUTP, respectively. After cDNA synthesis, the RNA was hydrolyzed using NaOH. The pH was neutralized with HCl and the labelled cDNA was purified by using QIAquick PCR purification spin columns (Qiagen). The efficiency of labelling was monitored spectrometricly on a Nanodrop (Isogen Life Science).
Labelled cDNA from control and sorbate treated samples of each time-point were mixed, dried and resuspended in hybridization buffer (25 mM HEPES, pH 8.0, 1 mM EDTA, 0.8 µg of yeast tRNA/µl, 3x SSC (0.3 M NaCl, 0.03 M sodium citrate pH 7.0) / 0.2 % (w/v) sodium dodecyl sulfate). Prior to hybridization, the DNA microarray slides were pre-hybridized by incubation in 2x SSPE (0.3 M sodium chloride, 0.02 M sodium hydrogen phosphate, 2 mM EDTA, pH 7.4) / 0.2 % SDS at 52°C for 1.5 hour. Next, the slides were washed in milliQ and dried by centrifugation. Hybridization was carried out in an automated slide processor (GE Healthcare) for 16 hours at 37°C. The slides were washed for 10 min in 1x SSC / 0.2 % SDS, next for 10 min in 0.1x SSC / 0.2 % SDS and finally for 4 min in 0.1x SSC. After a quick flush with isopropanol the arrays were dried under a nitrogen stream and immediately scanned on an Agilent G2505 scanner.

### Microarray data extraction and processing

Quantification of the hybridization signals from both Cy3- and Cy-5 channels and background subtractions was carried out with ArrayVision 6.1 software (Imaging Research Inc.). First, the pixels with density values that exceeded four median absolute deviations above the median were removed and the average of all pixels remaining in the spot was computed for each channel (the artefact-removed (ARM) density values). Second, the local background was calculated for each spot and subtracted from its ARM density value (resulting in the subtracted artefact-removed (sARM) density value). Spots with a signal-to-noise ratio (sARM divided by the standard deviation of the local background) smaller than 2.0 in both channels were excluded from further analysis. The remaining data was normalized in J-Express Pro 2.6 software (MolMine AS) using a global LOWESS (locally weighted scatterspot smoothing) algorithm (Yang YH, et al., 2002b Nucl. Acid Res., 30, e15). To avoid extreme intensity ratios, low intensity fluorescence data was floored at a value corresponding to a signal-to-noise ratio of 2.0. The data was averaged, log₂ transformed and missing values were replaced by the average of the closest values. Genes with more than two missing values in the time-series were omitted. To identify significantly expressed genes we applied an intensity-dependent method to identify differentially expressed genes (Yang *et al.,* 2002a). A sliding window of 50 genes was selected to calculate a Z-score from the local mean and standard deviation using the data in the R-I plot (log₁₀(Cy5*Cy3) vs. log₁₀(Cy5/Cy3). Genes more than 1.96 standard deviations away from the local average (IZI > 1.96) were considered differentially expressed. This corresponds to a confidence level of 95 %. Genes that showed significant expression at t = 0 min were excluded from further analysis, unless the gene showed opposite significant expression in at least one of the other time-points. After the processing of the microarray data 3909 genes remained for each time-point, of which 459 were found to be significantly expressed.

### Microarray data analysis

### i) Hierarchical clustering of significantly regulated genes

Hierarchical clustering (Eisen MB et al., (1998), Proc. Natl. Acad. Sci., 95: 14863-14868) of the significantly regulated genes was used to identify groups of genes with similar transcription profiles. In J-Express Pro 2.6 (MolMine AS) all 459 genes showing significant expression during potassium sorbate treatment were hierarchically clustered using the average linkage (WPGMA) clustering method and Euclidian distance metric.

### Example 2: Identification of a gene conferring resistance to sorbic acid in Bacillus subtilis by transposon mutagenesis

In order to study the molecular mechanism of the stress response of *B.subtilis* to weak organic acid, we tried to create mutants that are sensitive to sorbate. This was performed through a transposon mutagenesis using a mini-Tn*10* transposon system (Steinmetz and Richter, (1994), J. Bacteriol., 176: 1761-1763). This system is a very easy and convenient way to create single mutations in the genome of *B.subtilis* because the transposon inserts only once per cell into its genome. In this way 9 mutant libraries were created and two of them were screened for sorbate sensitive mutants.

### Results

### i. Isolation and restriction of pIC333 and transformation of B.subtilis

The mutagenesis was induced by using a transposon construct designed by Steinmetz and Richter (Steinmetz and Richter, 1994). The plasmid pIC333 (Steinmetz and Richter, 1994). containing all for the transposon mutagenesis required elements was stored in *E.coli* (XL1-BLUE). The plasmid was isolated from bacteria cells using a plasmid isolation kit (QIAGEN Benelux BV, Venlo, The Netherlands).

### Transformation of B.subtilis

Once the plasmid was isolated it needed to be transformed into the wild type *B.subtilis* PB2 as described in Material and Methods (ii). The transformants were grown on LB-plates containing 100µg/ml spectinomycin or 1µg/ml erythromycin to achieve a strict selection. The transformation efficiency was not calculated but appeared to be low.

Only those of the transformants that showed growth in both, erythromycin containing and spectinomycin containing liquid media were taken for the transposition.

### ii. Transposon mutagenesis

Transposon mutagenesis was performed as previously described (Material and Methods iii). Nine mutant banks (MB1-9) were created and stored at -80C in 15% glycerol to provide a stock.

### iii. Screening of Mutant Banks

MB#1 and MB#3 were chosen to be completely screened for interesting mutants according to the higher transposition efficiency compared to MB#2 (not shown). All mutants that did not grow on sorbate-containing plates or showed clearly reduced growth were considered as 'interesting mutants'.

### MB#1

On MB#1 first a test screen was performed on 15mM KSorbate. The concentrations between 15mM and 75mM were initially not tested, but it turned out that concentrations of 30mM and 40mM KSorbate did already reduce the growth of the wild type. The first screen showed very little success in terms of no detection of possible sensitive mutants, and further screens were carried out at a KS-concentration of 30mM. On the whole approximately 4500 different mutants were screened. The total yield of interesting mutants of Mutant Bank #1 was 76 (MB1.1-MB1.76).
All interesting mutants were grown in liquid LB-media, pH7.4, 80mM MOPS containing 100µg/ml spectinomycin and all showed growth and some of them showed growth in erythromycin containing media.

### MB#3:

On Mutant Bank #3 was only one screen with 30mM KSorbate at pH6.4 performed. This screen showed a yield of 58 interesting mutants (MB3.1-MB3.58) out of approximately 4250 screened mutants.

All interesting mutants were grown in liquid LB-media, pH6.4, 100µg/ml spectinomycin, and all mutants showed growth, some of them also showed growth in erythromycin containing media.

### iv. Micro Titer Plate - Experiments and Plating Assay

After the screening of the mutant banks, all interesting mutants were again checked to show a significant differentially behavior in the presence of sorbate. Therefore from all mutants a plating assay was made and growth was followed over 5 hours at 37°C while shaking in micro titer plates. The plating assay was incubated for about 24hours and pictures were taken. As a control the wild type PB2 was always included in the experiments and growth of the mutants compared to that of the wild type. Both experiments were started from the same culture in log-phase at an Optical Density (OD₆₀₀) of 0.2. Different 10fold dilutions (10° to 10⁻⁶) were performed on plates.

The majority of the interesting mutants were false positives and neither showed a significant differential behavior on sorbate plates nor in the growth curves.

From mutant bank #1 only 3 mutants (MB1.46, MB1.49, MB1.56) showed a clear differential growth, on the plates and in the micro titer plates (not shown).

There is a clear difference in the behavior observable when compared to the wild type. Also the pictures of the plating assay are consistent with the results of the MTP-experiments (not shown).

Of the 58 interesting mutants of MB#3 only six mutants (MB3.2, MB3.37, MB3.38, MB3.39, MB3.54 and MB3.58) showed a different behavior in the presence of sorbate (not shown), the rest turned out to be false positive. Just 2 of the six sensitive mutants showed reduced growth as clear as the mutants of MB#1; the remaining four did not show as clear differences when compared to the wild type.

All mutants show clearly a differential growth in early stationary phase, but in early exponential phase are the differences not really visible.

According to these results after 24hours, the mutants were considered as sorbate sensitive.

### v: DNA sequencing and gene identification

To sequence the DNA and to identify the gene that has been interrupted by the transposon mutagenesis, the genomic DNA had to be extracted using a kit (QIAGEN) and the purity and concentration was determined by absorbance measurement at wavelength of 260nm and 280nm (not shown).

The genomic DNA was analyzed on a 0.8% agarose gel before and after restriction with the restriction enzyme *Hin*dIII. After restriction the DNA was self-ligated using a Ready-To-Go DNA T4 Ligase (Amersham Bioscience, Piscataway, USA). The new created plasmids were transformed into *E.coli* XL1-BLUE competent cells. After selection for spectinomycin resistance all positive transformants were stored as plasmid stocks, because only the cells that took up the transposon-carrying plasmid were able to survive. The plasmids were isolated and digested by *Hin*dIII to estimate the size because at least one cut was expected. Further on it was restricted by *Bam*HI to check if the transposon was inserted (at least 2 cuts representing one 2.2kb fragment), by *Pst*I*, Bam*HI/*Pst*I and *Sph*I *(Pae*I*)* to distinguish between self-ligated plasmid and delivery vector pIC333 (not shown).

All isolated plasmids show a 2.2kb fragment when cut by *BamHI.* This is consistent with the expectation of a band representing the 2.2kb transposon (not shown).

All of the isolated plasmids show a different pattern than pIC333 when cut by *Sph*I and therefore seem not to be the delivery vector.

After this was confirmed either pure plasmid or plates with colonies could be sent to BaseClear Labservices (Leiden, The Netherlands) to be sequenced.

The self-ligated plasmids contain the transposon and parts of genomic DNA flanking it. 500 to 900nt were sequenced starting in the right (primer A) and left (primer B) end of the transposon sequence. The resulting sequences were now aligned to the genome of *B.subtilis* 168 using BLAST (http://genolist.pasteur.fr/SubtiList/).

The alignments of the nine sorbate sensitive mutants derived from Mutant Bank #1 and #3 were analyzed. Apparently four of the mutants had the same mutation with the same gene affected (*ymfM*). One of the four mutants (1.65) was renamed as ATB011.

### Discussion

The criteria determining whether a mutant is sorbate sensitive or not, are based upon reduced growth in the MTP-experiments and in the plating assay on different concentrations of KSorbate. In the growth curves differences are clearly visible that are also consistent with the results of the plates.

All mutants that showed the mutation in the gene *ymfM* behaved similar in the MTP-experiments as well as on the plates. They showed at low concentrations of sorbate already very reduced growth. This supports the idea, that the gene *ymfM* is important in coping with sorbate stress.

### Material and Methods

### i. Transformation of B.subtilis with pIC333

### Isolation of plasmid pIC333

The plasmid pIC333 was isolated from *E.coli* XL1-BLUE following the protocol for plasmid DNA-isolation (QIAGEN Benelux BV, Venlo, The Netherlands). A Gene map of the delivery vector pIC333 was disclosed in Steinmetz et al (Steinmetz and Richter, (1994), J. Bacteriol., 176: 1761-1763).
The isolated plasmid was checked by digestion by *Xba*I*, Bam*H1, *Eco*R1, *Sph*1 and *Hind*III according to the restriction sites on the plasmid. The samples were incubated for 90minutes at 37°C. The DNA-fragments were run on 0.8% agarose-gels and stained with ethidium bromide.

### Competent cells

*Bacillus subtilis* wild type strain PB2 provided by C.W. Price were used. This strain is also publicly available at the Bacillus Genetic Stock Centre via http://www.bgse.org/ using ID:1A776. To make PB2-cells competent they were grown on LB-agar over night at 37°C. Colonies were inoculated into liquid GCHE-medium (Kunst and Rapoport, (1995), Journal of Bacteriology, 177: 2403-2407) and grown overnight at 37°C, 250rpm. 1ml of over night culture was added to 10ml fresh GCHE-media and incubated at 37°C, 250rpm for 3 hours. After adding 11ml of pre-warmed GE-media (Kunst and Rapoport, 1995, Appendix) cells were again incubated at 37°C, 250rpm for 2 hours. Some competent cells were directly used for transformation; the remaining competent cells were stored in 15% glycerol stocks at -80°C.

### Transformation of B.subtilis

Six samples were prepared. Two samples containing no plasmid DNA, two containing 2µl plasmid DNA and two containing 10µl plasmid DNA. One of each were plated on LB-plates containing 1µg/ml erythromycin and LB-plates containing 100µg/ml spectinomycin. To each sample 200µl competent cells were added and incubated at 28°C, 250rpm for 30minutes. After adding 600µl of pre-warmed LB-medium, pH7.4, 80mM MOPS (3-[N-morpholono]propanesulfonic acid) the samples were again incubated at 28°C, 250rpm for 45minutes. After the final incubation, 100µl of each sample was plated on LB, pH7.4, 80mM MOPS, containing 1µg/ml erythromycin and 100µg/ml spectinomycin respectively. The rest of the samples were centrifuged for 10minutes at maximum speed. 600µl of the supernatants were discarded, the pellet gently resuspended in the remaining volume and each sample was plated on LB, pH7.4, 80mM MOPS, 1µg/ml erythromycin and 100µg/ml spectinomycin respectively. The petri dishes were incubated over night at 28°C.
For further transformations, four times 200µl competent cells were thawed and treated as previously described, using only 0µl and 10µl plasmid-DNA. Colonies of transformants were picked and grown over night in liquid LB, pH7.4, 80mM MOPS containing either 1µg/ml erythromycin or 100µg/ml spectinomycin. Only cultures that grew in both spectinomycin-containing media and in erythromycin-containing media were used for the mutagenesis.

### ii. Transposon Mutagenesis

### Transposon Mutagenesis System pIC333

The system used for mutagenesis is the mini-Tn*10*-OS (Steinmetz and Richter, 1994) inserted in the delivery vector pIC333. It carries the spectinomycin resistance gene and a pUC origin of replication for *E.coli.* The two ends of it determine the size of 2.2kb. There is a third 'unwanted' end of the transposon right after the for the, transposition necessary enzyme, transposase A, which is normally located outside the transposon on the plasmid. Additionally, the plasmid contains an erythromycin resistance and a temperature sensitive origin of replication that is not active above 30°C. A temperature shift from 28°C up to 37°C induces the transposition and the insertion of the transposable element into the host genome. At 37°C the plasmid is not able to replicate anymore and only the cells can survive that has inserted the transposon into the genome.

### Transposition

An over night culture of transformants that grew on erythromycin and spectinomycin was diluted 100fold in LB, pH7.4, and 80mM MOPS and incubated at 28°C, 250rpm. To perform the mutagenesis, after 3hours the temperature was increased to 37°C and the samples were again incubated for another 4hours at 250rpm. Cultures were stored in 15% glycerol stocks at -80°C.

With this method, nine Mutant Banks (MB#1-MB#9) were created to have a stock if in case some do not show the right statistical properties.

### iii. Screen of Mutant Banks

The mutant banks (MB#1 and MB#3) were diluted to an average amount of 50 to 100cells per plate and plated on LB, pH7.4, 80mM MOPS containing 100µg/ml spectinomycin. After over night incubation at 37°C, cells were transferred to LB-plates, pH6.4, 80mM MOPS containing 30mM KSorbate, 100µg/ml spectinomycin, and LB-plates, pH7.4, 80mM MOPS containing 1µg/ml erythromycin, and MM-media-plates, pH7.4, (5mM glucose/10mM NH₄Cl/10MM glutamate) containing 100µg/ml spectinomycin. Finally, the colonies were also transferred to LB-plates, pH7.4, 80mM MOPS containing 100µg/ml spectinomycin by replica plating using sterile velvets to transfer the cells. Plates were incubated at 37°C over night. Replica plating to LB-plates containing erythromycin and MM-plates was to determine percentage of mutants still containing the plasmid and the amount of auxotrophic mutants as previously described. The transfer of cells to the final plates just containing 100µg/ml spectinomycin was to check the transfer of cells at all. The sorbate-containing plates were compared to the original plate (without sorbate) and cells that showed less growth or no growth at all were considered as "interesting mutants". All mutants were grown in liquid LB-media, pH7.4, 80mM MOPS, containing 100µg/ml spectinomycin and liquid LB-media containing 1µg/ml erythromycin to control the loss of the plasmid pIC333. All mutants were stored at -80°C in 15% glycerol stocks.

### iv. Monitoring the growth of interesting mutants in Micro-Titer-Plate experiments and on plates containing different concentrations of KSsorbate (Plating Assay)

To check sorbate-sensitivity all interesting mutants were checked on plates containing different concentrations of KSorbate and also growth was monitored on micro titer plates.

### Plating assay

The mutants and the wild type PB2 as a control were grown over night in liquid LB-media, pH6.4, 80mM MOPS and 100µg/ml spectinomycin (for the mutants only) at 37°C and 250rpm. The cultures were diluted to an OD₆₀₀ of 0.2-0.25, grown to OD₆₀₀=0.5 and again diluted to an OD₆₀₀=0.020-0.025. At log-phase (OD₆₀₀=0.2) a 200µl sample was taken, 10°fold to 10⁻⁶fold diluted and ~5µl plated on the same LB-plates, pH6.4, 80mM MOPS containing 0mM, 15mM, 30mM and 40mM KSorbate. After 24h of incubation at 37°C pictures of the plates were taken.

### Micro Titer Plate experiment

To monitor the growth of the interesting mutants, the remaining cultures after plating were further grown to an OD₆₀₀=0.4 and 2x diluted in a 96well micro titer plate containing different concentrations of KSorbate (0mM, 15mM, 30mM, 40mM, 50mM, 75mM KSorbate). Growth was followed over 5hours at 37°C and 250rpm.

Mutants that showed less growth compared to the wild type PB2 were considered as sorbate sensitive mutants.

### v. DNA-isolation, digestion, self-ligation and cloning into E.coli

### Genomic DNA-isolation

The mutants were plated and grown in liquid LB-media containing 100µg/ml spectinomycin. The OD₆₀₀ was measured and an appropriate volume of the over night culture was taken to reach the recommended cell density. The DNA was isolated according to the protocol of the kit for genomic DNA extraction (QIAGEN Benelux BV, Venlo, The Netherlands). The DNA was precipitated in isopropanol, spinned down, washed in 70% ice cold ethanol and dissolved in 200µl TE, pH8.0 over night at room temperature and 250rpm and stored at 4°C. The purity (ratio of A₂₆₀/A₂₈₀ = 1.7-1.9) and the concentration (A260 = 0.5-1.0 equals 25-50ng DNA/µl) were determined by absorbance measurement at 260nm (A₂₆₀) and 280nm (A₂₈₀) wavelength and a 0.8% agarose gel was run and stained with ethidium bromide.

### Digestion of genomic DNA

A sample of 250ng-3.5µg DNA was digested by *HindIII,* which is a restriction enzyme that does not cut in the transposon sequence. The restriction was checked on a 0.8% agarose gel and stained with ethidium bromide.

### Ligation

To purify the DNA from the restriction mixture, the DNA was precipitated by adding 1/10vol 3M NaAC, pH5.2 and 3vol 100% ethanol. The reaction was incubated for 30minutes at -80°C, centrifuged 15minutes at 4°C and >5000g, washed with 70% ethanol, centrifuged for another 15 minutes at 4°C and >5000g and resuspended in 50µl TE, pH8.0.
A total amount of ~95ng and -250ng of DNA was self-ligated using a Ready-To-Go DNA T4 Ligase (Amersham Bioscience, Piscataway, USA). The samples were incubated for 5 minutes at room temperature, mixed and gently collected at the bottom of the reaction tube. After 45 minutes at 16°C the reaction was inactivated at 70°C for 10 minutes.

### Transformation of E.coli XL1-BLUE

After inactivation of the ligation the DNA was directly used for transformation of highly competent *E.coli* XL1-BLUE (Stratagene, Amsterdam, The Netherlands) cells. 2µl and the remaining volume of the ligation samples were added to 50µl competent cells each. Also 1µl test plasmid pUC19 was added to 25µl competent cells as positive control and a 25µl sample competent cells without DNA served as negative control. The samples were incubated for 30 minutes on ice, 2 minutes at 42C and again 5 minutes on ice. After adding 1ml of pre-warmed LB-media the cells were incubated for one hour at 37C, 250rpm. 100µl of the transformants and the negative control were plated on pre-warmed LB-plates, 100µg/ml spectinomycin and 100µl and 10µl of the positive control was plated on LB, 100µg/ml ampicillin. All samples except the positive control were spinned down, 900µl of the supernatant discarded, the pellets resuspended in the remaining volume and plated on LB, 100µg/ml spectinomycin. The plates were incubated over night at 37C. To calculate transformation efficiency the cells of the positive control were counted. After growing transformants over night in 5ml liquid LB, 100µg/ml spectinomycin, 15% glycerol stocks were made and cells of 2ml cultures were harvested by centrifugation for 5 minutes at full speed. The plasmid DNA was isolated following the protocol of plasmid DNA Isolation (QIAGEN Benelux BV, Venlo, The Netherlands) and dissolved in 100µl TE, pH8.0.
To control the plasmid and to determine its size, the DNA was digested by *Bam*HI, *Hin*dIII, *Pst*I, *Bam*HI/*Pst*I and *Sph*I *(Pae*I*)* for 90 minutes and run on a 0.8% agarose gel stained with ethidium bromide.

### vi. DNA-sequencing and gene identification

For sequencing of the self ligated plasmids, two 21nucleotide (table 2) long primers that are complementary to the right and left end of the transposon were ordered.

**Table 2: Used primers of left and right end of transposon**

| Primer | Sequence |
|---|---|
| pIC333-right (A) SEQ ID NO:7 | 5'-GGCCGATTCATTAATGCAGGG-3' |
| pIC333-left (B) SEQ ID NO: 8 | 5'-CGATATTCACGGTTTACCCAC-3' |

The primers and the samples (pure plasmid or plates with colonies) were sent to be sequenced by BaseClear Labservices (Leiden, The Netherlands).
The results were aligned to the genome of *B.subtilis* 168 using BLAST at the 'Subtilist'-server (http://genolist.pasteur.fr/SubtiList/) in order to identify the mutated gene.

## Claims

1. A screening method for the identification of a preservative compound, which method comprises:
(a) contacting a potential preservative compound, preferably in the presence of a weak acid with a biological target, said biological target comprising a polypeptide selected from the group consisting of:
(i) YhcA having an amino acid sequence which has at least about 16% identity with the amino acid sequence of SEQ ID NO:1,
(ii) YmfM having an amino acid sequence which has at least about 28% identity with the amino acid sequence of SEQ ID NO:2 and,
(iii) a phenolic acid decarboxylase PadC having an amino acid sequence which has at least about 45% identity with the amino acid sequence of SEQ ID NO:3, and subsequently,
(b) identifying an effect such as reduction of a polypeptide activity which the potential preservative compound has on the biological target; whereby the effect is indicative for preservative activity of the potential preservative compound.

2. The screening method according to claim 1, wherein the contacting step is carried out *in vivo* and the biological target is a cell.

3. The screening method according to claim 1, wherein the contacting step is carried out *in vitro* and the polypeptide is as defined in (iii).

4. The screening method according to any one of claims 1 to 3, wherein the effect is reduction of a polypeptide activity, preferably decarboxylase activity.

5. The screening method according to claim 2 or 4, wherein the effect is reduction of the intracellulair pH, and/or reduction of cell growth and/or cell death.

6. The screening method according to claim 4 or 5, wherein a reduction of a polypeptide activity of at least 10% in the presence of the potential preservative compound compared to the activity of the polypeptide in the absence of said potential preservative compound is indicative for preservative activity of the potential preservative compound.

7. The screening method according to claim 5 or 6, wherein a reduction of the intracellulair pH in the presence of the potential preservative compound of at least 0.1 unit compared to the intracellulair pH in the absence of said potential preservative compound is indicative for preservative activity of the potential preservative compound.

8. The screening method according to any one of claims 5 to 7, wherein a reduction of the cell growth in the presence of the potential preservative compound of at least 10% compared to the cell growth in the absence of said potential preservative compound is indicative for preservative activity of the potential preservative compound.

9. The screening method according to any one of the preceding claims, wherein the effect is determined using a microtitre plate assay based on radioactivity, fluorescence or by HPLC.

10. A polypeptide YhcA having an amino acid sequence which has at least 41 % identity with the amino acid sequence of SEQ ID NO:1.

11. The polypeptide according to claim 10, wherein the polypeptide has the amino acid sequence of SEQ ID NO:1.

12. The polypeptide according to claim 10 or 11, wherein the polypeptide is obtained from a bacterial strain, preferably a *Bacillus* species, more preferably a *Bacillus subtilis* strain.

13. A polypeptide YmfM having an amino acid sequence which has at least 52% identity with the amino acid sequence of SEQ ID NO:2.

14. The polypeptide according to claim 13, wherein the polypeptide has the amino acid sequence of SEQ ID NO:2.

15. The polypeptide according to claim 13 or 14, wherein the polypeptide is obtained from a bacterial strain, preferably a *Bacillus* species, more preferably a *Bacillus subtilis* strain.

16. A polypeptide PadC having phenolic acid decarboxylase activity, wherein said polypeptide has an amino acid sequence which has at least 98% identity with the amino acid sequence of SEQ ID NO:3.

17. The polypeptide according to claim 8, wherein the polypeptide has the amino acid sequence of SEQ ID NO:3.

18. The polypeptide according to claim 16 or 17, wherein the polypeptide is obtained from a bacterial strain, preferably a *Bacillus* species, more preferably a *Bacillus subtilis* strain.

19. A nucleic acid construct comprising the nucleic acid sequence coding for a polypeptide according to any one of claims 10 to 18.

20. An expression vector comprising the nucleic acid construct of claim 19.

21. A host cell comprising the nucleic acid construct of claim 19 or the expression vector of claim 20.


SEQUENCE LISTING

<110>  Unilever N.V.

<120>  Screening method for the identification of a preservative

<130>  F7944(V)GT/CO

<140>  EP06126658.1
<141>  2006-12-20

<160>  10

<170>  PatentIn version 3.3

<210>  1
<211>  532
<212>  PRT
<213>  Bacillus subtilis

<400>  1

Met Ala Thr Gly Lys Glu Lys Asn Ala Lys Asn Pro Met Ser Leu Leu
1               5                   10                  15

Ile Val Leu Met Ala Gly Leu Phe Leu Ala Ile Leu Asn Gln Thr Leu
            20                  25                  30

Leu Asn Val Ala Met Pro His Leu Met Thr Glu Phe Gly Val Ser Ala
        35                  40                  45

Thr Thr Ile Gln Trp Leu Thr Thr Gly Tyr Met Leu Val Asn Gly Val
    50                  55                  60

Leu Ile Pro Leu Ser Ala Phe Leu Ile Thr Arg Phe Gly Gln Arg Ser
65                  70                  75                  80

Leu Phe Leu Val Ala Met Phe Cys Phe Thr Leu Gly Thr Leu Val Cys
                85                  90                  95

Gly Ile Ala Pro Asn Phe Ser Thr Met Leu Ile Gly Arg Leu Ile Gln
            100                 105                 110

Ala Val Gly Gly Gly Ile Leu Gln Pro Leu Val Met Thr Thr Ile Leu
        115                 120                 125

Leu Ile Phe Pro Pro Glu Ser Arg Gly Lys Gly Met Gly Ile Phe Gly
    130                 135                 140

Leu Ala Met Met Phe Ala Pro Ala Val Gly Pro Thr Leu Ser Gly Trp
145                 150                 155                 160

Ile Ile Glu His Tyr Thr Trp Arg Ile Met Phe Tyr Gly Leu Val Pro
                165                 170                 175

Ile Gly Ala Ile Val Ile Ile Val Ala Phe Phe Ile Phe Lys Asn Met
            180                 185                 190

Val Glu Pro Gln Lys Ile Lys Leu Asp Thr Leu Gly Ala Ile Leu Ser
        195                 200                 205

Ile Val Gly Phe Ala Ser Leu Leu Tyr Gly Val Ser Glu Ala Gly Ser
    210                 215                 220

Asp Gly Trp Thr Asp Pro Ile Val Leu Ser Thr Val Ile Ile Gly Ala
225                 230                 235                 240

Ile Ala Ile Val Ala Phe Val Val Gln Gln Leu Arg His Asp Asp Pro
                245                 250                 255

Met Leu Asp Phe Arg Val Phe Lys Tyr Asp Ile Phe Ser Leu Ser Ser
            260                 265                 270

Val Ile Asn Ile Ile Ile Thr Val Ala Leu Tyr Thr Gly Met Phe Leu
        275                 280                 285

Leu Pro Ile Tyr Leu Gln Asn Leu Val Gly Phe Thr Ala Leu Gln Ser
    290                 295                 300

Gly Leu Leu Leu Leu Pro Gly Ala Ile Val Met Leu Ile Met Ser Pro
305                 310                 315                 320

Ile Ser Gly Ile Leu Phe Asp Lys Phe Gly Pro Arg Pro Leu Ala Ile
                325                 330                 335

Ile Gly Leu Leu Val Thr Val Val Thr Thr Tyr Gln Tyr Thr Gln Leu
            340                 345                 350

Thr Ile Asp Thr Pro Tyr Thr His Ile Met Leu Ile Tyr Ser Ile Arg
        355                 360                 365

Ala Phe Gly Met Ser Leu Leu Met Met Pro Val Met Thr Ala Gly Met
    370                 375                 380

Asn Gln Leu Pro Ala Arg Leu Asn Ser His Gly Thr Ala Met Ser Asn
385                 390                 395                 400

Thr Leu Arg Gln Ile Ser Gly Ser Ile Gly Thr Ser Leu Ile Thr Thr
                405                 410                 415

Ile Tyr Thr Asn Arg Thr Thr Phe His Tyr Ser Gln Ile Ala Asp Lys
            420                 425                 430

Thr Ser Thr Ala Asp Pro Asn Phe Leu His Ala Phe Gln Asn Ala Val
        435                 440                 445

Ser Asn Leu Met Val Asn Met Asn Val Ser Tyr Asp Thr Ala Lys Thr
    450                 455                 460

Tyr Val Tyr Ser His Ile Tyr Lys His Ala Ser Leu Asp Ser Asn Val
465                 470                 475                 480

Met Gly Ile Asn Asp Ala Phe Met Trp Ala Thr Leu Phe Cys Val Ala
                485                 490                 495

Gly Leu Ile Leu Ser Ile Phe Leu Arg Asp Val Arg Lys Asp Lys Leu
            500                 505                 510

Arg Lys Lys Lys Lys Glu Glu Leu Ser Leu Leu Pro Ala Pro Lys Glu
        515                 520                 525

Ala Lys Glu Ser
    530

<210>  2
<211>  213
<212>  PRT
<213>  Bacillus subtilis

<400>  2

Met Ser Leu Asp Asp Leu Gln Ala Ala Thr Lys Ile Gln Lys Arg Tyr
1               5                   10                  15

Leu Thr Ala Leu Glu Glu Gly Asn Tyr Asp Ile Ile Pro Gly Lys Val

            20                  25                  30

Leu Cys Ser His Ser Ser Ser Asn Met Gln Lys Pro Leu Ala Asn Asp
        35                  40                  45

Ala Asp Gln Leu Phe Glu Glu His Lys Lys Asp Ile Pro Asn Thr Tyr
    50                  55                  60

His Asp Asp Val Ser Glu Lys Ile Ser Gly Met Asn Leu Gln Lys Glu
65                  70                  75                  80

Met Pro Lys Pro Ala Ser Lys Ala Leu Glu Leu Leu Pro Thr Ile Leu
                85                  90                  95

Val Ile Leu Gly Val Ile Val Val Ile Ala Ile Val Tyr Ala Ile Ile
            100                 105                 110

Gln Phe Gly Asn His Lys Asn Ser Asp Asp His Asn Ala Gly Ser Glu
        115                 120                 125

Lys Ala Ile Thr Gln Ser Glu Ser Lys Tyr Glu Ile Pro Lys Asp Ser
    130                 135                 140

Thr Leu Lys Glu Asn Gln Asn Asn Ser Ser Glu Lys Glu Thr Asp Thr
145                 150                 155                 160

Lys Lys Glu Thr Lys Glu Asn Glu Asp Lys Lys Lys Glu Asn Asp Ser
                165                 170                 175

Glu Lys Leu Glu Ile Asn Ala Ala Gly Thr Glu Gly Ser Leu Thr Thr
            180                 185                 190

Tyr Glu Val Ser Gly Ala Asp Lys Ile Asp Leu Glu Leu Lys Ala Ser
        195                 200                 205

Asp Ser Ser Trp Ile
    210

<210>  3
<211>  161
<212>  PRT
<213>  Bacillus subtilis

<400>  3

<210> 4
<211> 1596
<212> DNA
<213> Bacillus subtilis

<400> 4
atggctacag gcaaagaaaa aaatgcaaaa aaccctatgt cattgctgat tgtgctaatg 60

gcgggtttat ttttggcaat tctaaaccaa acactgctta atgttgcaat gccgcactta 120

atgactgaat ttggcgtaag tgcaacgacg attcagtggc tgacaacagg ttacatgctt 180

gttaacggtg tattgattcc gttatctgct ttcctgatta cacgatttgg ccagcggagt 240

ctctttctcg tcgcgatgtt ttgttttacg ctcggtacgt tagtctgcgg tatcgcacct 300

aacttctcta caatgctgat cggccgtttg attcaggccg tgggcggagg tatcttgcag 360

ccgctcgtta tgacaaccat tctgttaatt ttcccgcctg aaagcagggg gaaaggaatg 420

gggattttcg ggctagcgat gatgtttgct ccagcggtcg gcccgactct ttccgggtgg 480

attattgagc attatacatg gcgcattatg ttttacggac ttgtgccgat cggcgcaatt 540

gttatcattg tggctttctt catctttaaa aatatggtgg agccgcaaaa aatcaaactc 600

gatactcttg gcgcaatcct ctcgattgtc ggattcgcct ccttattata cggagtcagt 660

gaagccggat ctgacggctg gaccgacccg attgtattgt ctacagttat tatcggagcc 720

attgcaattg ttgcttttgt tgttcagcaa ttgcgtcatg atgatccgat gcttgatttc 780

agagtgttta aatatgacat tttctcactc tcgagtgtca ttaatatcat tattacagtg 840

gcactttaca ctggtatgtt ccttttaccg atttatctgc aaaacctggt cggctttacg 900

gcgcttcaat ccggtctgct tctgcttccg ggtgcgatcg ttatgctgat tatgtcaccg 960

atctcaggta ttttgttcga taaattcgga ccgagaccgc ttgcgattat cgggcttctc 1020

gtgacagtgg taacaacgta tcaatacacg caattgacaa ttgatacacc atatacacac 1080

atcatgctga tctattcaat tcgtgcattt ggtatgtctc tcttaatgat gccagtcatg 1140

acagccggga tgaaccagct tcctgcccgt ctgaacagcc acggaacggc gatgagtaac 1200

acgcttcgcc aaatcagcgg atcaatcgga acaagtttaa ttacgacgat ttatacaaac 1260

cggacgacat tccattattc acaaattgca gataaaacaa gcacggcgga cccgaacttc 1320

ctccatgcgt tccaaaatgc agtttcgaat ttaatggtga atatgaatgt gtcttatgat 1380

acggcgaaaa catatgtgta ctcacatatt tataaacacg catcactcga ttccaatgtc 1440

atggggataa atgatgcctt tatgtgggca accttattct gtgtagcagg tttgatttta 1500

agtatcttcc tgcgtgacgt cagaaaagat aaattgcgta agaagaaaaa agaggagctt 1560

tcattacttc ctgctccaaa agaagctaag gaatct 1596

<210> 5

<211> 639

<212> DNA

<213> Bacillus subtilis

<400> 5

atgtcattgg atgatctcca agcggcaaca aagattcaaa aaaggtattt aaccgccttg 60

gaggaaggaa actatgacat tattccgggg aaagttttat gttcgcattc atcaagcaat 120

atgcagaagc cgttggccaa tgatgcggat cagctgtttg aggagcataa aaaagatatc 180

cctaatacgt atcatgatga tgtgtctgaa aagatctccg gcatgaatct tcaaaaggaa 240

atgccaaagc cagcatctaa ggcgctggag ctgctgccaa caatacttgt tattctcggc 300

gtgattgtcg tgattgccat tgtgtacgcg atcattcaat ttggcaatca caaaaacagt 360

gatgatcaca atgcgggatc agaaaaagca attacgcaaa gtgagagcaa gtacgaaatc 420

cctaaagatt caacgctgaa agagaatcaa aataacagct ctgaaaaaga gacagacact 480

aaaaaagaaa cgaaagaaaa cgaagataag aaaaaagaaa atgacagtga gaagctggag 540

attaacgctg ctggcactga aggatcgtta acgacttatg aagtgtccgg cgctgataaa 600

atcgacctgg aactgaaggc atcagacagc tcctggatt 639

<210> 6
<211> 483
<212> DNA
<213> Bacillus subtilis

<400> 6
atggaaaact ttatcggaag ccacatgatt tatacgtatg aaaacggatg ggaatacgag 60

atttatatta aaaacgacca tacaattgat tatagaattc atagcggaat ggttgccgga 120

cgctgggttc gagatcagga agtgaatatt gtcaaactga cagaaggcgt atataaagtg 180

tcttggacag agccgactgg cacggatgtt tcattaaact ttatgccaaa tgaaaaacgc    240

atgcatggca ttattttctt cccgaaatgg gtgcatgaac atcctgaaat tacggtttgc    300

taccaaaatg accacattga tttgatgaaa gaatcccgcg aaaaatatga aacgtatcca    360

aaatacgttg tacctgaatt tgcggaaatt acatttctga aaaatgaagg agtcgacaac    420

gaagaagtga tttcgaaggc tccttatgag ggaatgacag acgatattcg cgcgggaaga    480

tta                                                                  483

<210>  7
<211>  21
<212>  DNA
<213>  artificial

<220>
<223>  primer

<400>  7
ggccgattca ttaatgcagg g                                               21

<210>  8
<211>  21
<212>  DNA
<213>  artificial

<220>
<223>  primer

<400>  8

cgatattcac ggtttaccca c                                                21

<210>  9
<211>  246
<212>  PRT
<213>  Aequorea victoria

<400>  9

Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Thr Phe
        35                  40                  45

Ile Cys Thr Thr Gly Lys Leu Pro Val Pro Trp Pro Thr Leu Val Thr
    50                  55                  60

Thr Phe Ser Tyr Gly Val Gln Cys Phe Ser Arg Tyr Pro Asp His Met
65                  70                  75                  80

Lys Arg His Asp Phe Phe Lys Ser Ala Met Pro Glu Gly Tyr Val Gln
                85                  90                  95

Glu Arg Thr Ile Phe Phe Lys Asp Asp Gly Phe Thr Asn Tyr Lys Thr
            100                 105                 110

Arg Ala Glu Val Lys Phe Glu Gly Asp Thr Leu Val Asn Arg Ile Glu
        115                 120                 125

Leu Lys Gly Ile Asp Phe Lys Asp Asp Gly Asn Ile Leu Gly His Lys
    130                 135                 140

Leu Glu Tyr Asn Tyr Asn Glu His Leu Val Tyr Ile Met Ala Asp Lys
145                 150                 155                 160

Gln Lys Asn Gly Thr Lys Ala Phe Thr Ile Phe Gln Val His His Asn
                165                 170                 175

Ile Glu Asp Gly Gly Val Gln Leu Ala Asp His Tyr Gln Gln Asn Thr
            180                 185                 190

Pro Ile Gly Asp Gly Pro Val Leu Leu Pro Asp Asn His Tyr Leu His
        195                 200                 205

Thr Gln Ser Ala Leu Ser Lys Asp Pro Asn Glu Lys Arg Asp His Met
    210                 215                 220

Val Phe Leu Glu Phe Thr Phe Val Thr Ala Ala Gly Ile Thr His Gly
225                 230                 235                 240

Met Asp Glu Leu Tyr Lys
                245

<210>  10
<211>  717
<212>  DNA
<213>  Aequorea victoria

<400>  10
atgagtaaag gagaagaact tttcactgga gttgtcccaa ttcttgttga attagatggt      60

gatgttaatg ggcacaaatt ttctgtcagt ggagagggtg aaggtgatgc aacatacgga     120

aaacttaccc ttaaatttat ttgcactact ggaaaactac ctgttccatg gccaacactt     180

gtcactactt tctcttatgg tgttcaatgc ttttcaagat acccagatca tatgaaacgg     240

catgactttt tcaagagtgc catgcccgaa ggttatgtac aggaaagaac tatatttttc     300

aaagatgacg ggaactacaa gacacgtgct gaagtcaagt ttgaaggtga tacccttgtt     360

aatagaatcg agttaaaagg tattgatttt aaagatgatg gaaacattct tggacacaaa     420

ttggaataca actataacga gcacttggtg tacatcatgg cagacaaaca aaagaatggt     480

accaaagcta tctttcaagt tcaccacaac attgaagatg gaggcgttca actagcagac     540

cattatcaac aaaatactcc aattggcgat ggccctgtcc ttttaccaga caaccattac     600

ctgcacacac aatctgccct ttcgaaagat cccaacgaaa agagagacca catggtcttt     660

cttgagtttg taacagctgc tgggattaca catggcatgg atgaactata caaataa 717

SEQUENCE LISTING

<110>  Unilever N.V.

<120>  Screening method for the identification of a preservative

<130>  A6010291

<160>  10

<170>  PatentIn version 3.3

<210>  1
<211>  532
<212>  PRT
<213>  Bacillus subtilis

<400>  1

Met Ala Thr Gly Lys Glu Lys Asn Ala Lys Asn Pro Met Ser Leu Leu
1               5                   10                  15

Ile Val Leu Met Ala Gly Leu Phe Leu Ala Ile Leu Asn Gln Thr Leu
            20                  25                  30

Leu Asn Val Ala Met Pro His Leu Met Thr Glu Phe Gly Val Ser Ala
        35                  40                  45

Thr Thr Ile Gln Trp Leu Thr Thr Gly Tyr Met Leu Val Asn Gly Val
    50                  55                  60

Leu Ile Pro Leu Ser Ala Phe Leu Ile Thr Arg Phe Gly Gln Arg Ser
65                  70                  75                  80

Leu Phe Leu Val Ala Met Phe Cys Phe Thr Leu Gly Thr Leu Val Cys
                85                  90                  95

Gly Ile Ala Pro Asn Phe Ser Thr Met Leu Ile Gly Arg Leu Ile Gln
            100                 105                 110

Ala Val Gly Gly Gly Ile Leu Gln Pro Leu Val Met Thr Thr Ile Leu
        115                 120                 125

Leu Ile Phe Pro Pro Glu Ser Arg Gly Lys Gly Met Gly Ile Phe Gly
    130                 135                 140

Leu Ala Met Met Phe Ala Pro Ala Val Gly Pro Thr Leu Ser Gly Trp
145                 150                 155                 160

Ile Ile Glu His Tyr Thr Trp Arg Ile Met Phe Tyr Gly Leu Val Pro
                165                 170                 175

Ile Gly Ala Ile Val Ile Ile Val Ala Phe Phe Ile Phe Lys Asn Met
            180                 185                 190

Val Glu Pro Gln Lys Ile Lys Leu Asp Thr Leu Gly Ala Ile Leu Ser
        195                 200                 205

Ile Val Gly Phe Ala Ser Leu Leu Tyr Gly Val Ser Glu Ala Gly Ser
    210                 215                 220

Asp Gly Trp Thr Asp Pro Ile Val Leu Ser Thr Val Ile Ile Gly Ala
225                 230                 235                 240

Ile Ala Ile Val Ala Phe Val Val Gln Gln Leu Arg His Asp Asp Pro
                245                 250                 255

Met Leu Asp Phe Arg Val Phe Lys Tyr Asp Ile Phe Ser Leu Ser Ser
            260                 265                 270

Val Ile Asn Ile Ile Ile Thr Val Ala Leu Tyr Thr Gly Met Phe Leu
        275                 280                 285

Leu Pro Ile Tyr Leu Gln Asn Leu Val Gly Phe Thr Ala Leu Gln Ser
    290                 295                 300

Gly Leu Leu Leu Leu Pro Gly Ala Ile Val Met Leu Ile Met Ser Pro
305                 310                 315                 320

Ile Ser Gly Ile Leu Phe Asp Lys Phe Gly Pro Arg Pro Leu Ala Ile
                325                 330                 335

Ile Gly Leu Leu Val Thr Val Val Thr Thr Tyr Gln Tyr Thr Gln Leu
            340                 345                 350

Thr Ile Asp Thr Pro Tyr Thr His Ile Met Leu Ile Tyr Ser Ile Arg
        355                 360                 365

Ala Phe Gly Met Ser Leu Leu Met Met Pro Val Met Thr Ala Gly Met
    370                 375                 380

Asn Gln Leu Pro Ala Arg Leu Asn Ser His Gly Thr Ala Met Ser Asn
385                 390                 395                 400

Thr Leu Arg Gln Ile Ser Gly Ser Ile Gly Thr Ser Leu Ile Thr Thr
                405                 410                 415

Ile Tyr Thr Asn Arg Thr Thr Phe His Tyr Ser Gln Ile Ala Asp Lys
            420                 425                 430

Thr Ser Thr Ala Asp Pro Asn Phe Leu His Ala Phe Gln Asn Ala Val
        435                 440                 445

Ser Asn Leu Met Val Asn Met Asn Val Ser Tyr Asp Thr Ala Lys Thr
    450                 455                 460

Tyr Val Tyr Ser His Ile Tyr Lys His Ala Ser Leu Asp Ser Asn Val
465                 470                 475                 480

Met Gly Ile Asn Asp Ala Phe Met Trp Ala Thr Leu Phe Cys Val Ala
                485                 490                 495

Gly Leu Ile Leu Ser Ile Phe Leu Arg Asp Val Arg Lys Asp Lys Leu
            500                 505                 510

Arg Lys Lys Lys Lys Glu Glu Leu Ser Leu Leu Pro Ala Pro Lys Glu
        515                 520                 525

Ala Lys Glu Ser
    530

<210>  2
<211>  213
<212>  PRT
<213>  Bacillus subtilis

<400>  2

Met Ser Leu Asp Asp Leu Gln Ala Ala Thr Lys Ile Gln Lys Arg Tyr
1               5                   10                  15

Leu Thr Ala Leu Glu Glu Gly Asn Tyr Asp Ile Ile Pro Gly Lys Val
            20                  25                  30

Leu Cys Ser His Ser Ser Ser Asn Met Gln Lys Pro Leu Ala Asn Asp
        35                  40                  45

Ala Asp Gln Leu Phe Glu Glu His Lys Lys Asp Ile Pro Asn Thr Tyr
    50                  55                  60

His Asp Asp Val Ser Glu Lys Ile Ser Gly Met Asn Leu Gln Lys Glu
65                  70                  75                  80

Met Pro Lys Pro Ala Ser Lys Ala Leu Glu Leu Leu Pro Thr Ile Leu

85                  90                  95

Val Ile Leu Gly Val Ile Val Val Ile Ala Ile Val Tyr Ala Ile Ile
            100                 105                 110

Gln Phe Gly Asn His Lys Asn Ser Asp Asp His Asn Ala Gly Ser Glu
        115                 120                 125

Lys Ala Ile Thr Gln Ser Glu Ser Lys Tyr Glu Ile Pro Lys Asp Ser
    130                 135                 140

Thr Leu Lys Glu Asn Gln Asn Asn Ser Ser Glu Lys Glu Thr Asp Thr
145                 150                 155                 160

Lys Lys Glu Thr Lys Glu Asn Glu Asp Lys Lys Lys Glu Asn Asp Ser
                165                 170                 175

Glu Lys Leu Glu Ile Asn Ala Ala Gly Thr Glu Gly Ser Leu Thr Thr
            180                 185                 190

Tyr Glu Val Ser Gly Ala Asp Lys Ile Asp Leu Glu Leu Lys Ala Ser
        195                 200                 205

Asp Ser Ser Trp Ile
    210

<210>  3
<211>  161
<212>  PRT
<213>  Bacillus subtilis

<400>  3

<210>  4
<211>  1596
<212>  DNA
<213>  Bacillus subtilis

<400>  4
atggctacag gcaaagaaaa aaatgcaaaa aaccctatgt cattgctgat tgtgctaatg      60

gcgggtttat ttttggcaat tctaaaccaa acactgctta atgttgcaat gccgcactta     120

atgactgaat ttggcgtaag tgcaacgacg attcagtggc tgacaacagg ttacatgctt     180

gttaacggtg tattgattcc gttatctgct ttcctgatta cacgatttgg ccagcggagt     240

ctctttctcg tcgcgatgtt ttgttttacg ctcggtacgt tagtctgcgg tatcgcacct     300

aacttctcta caatgctgat cggccgtttg attcaggccg tgggcggagg tatcttgcag     360

ccgctcgtta tgacaaccat tctgttaatt ttcccgcctg aaagcagggg gaaaggaatg     420

gggattttcg ggctagcgat gatgtttgct ccagcggtcg gcccgactct ttccgggtgg     480

attattgagc attatacatg gcgcattatg ttttacggac ttgtgccgat cggcgcaatt     540

gttatcattg tggctttctt catctttaaa aatatggtgg agccgcaaaa aatcaaactc     600

gatactcttg gcgcaatcct ctcgattgtc ggattcgcct ccttattata cggagtcagt     660

gaagccggat ctgacggctg gaccgacccg attgtattgt ctacagttat tatcggagcc     720

attgcaattg ttgcttttgt tgttcagcaa ttgcgtcatg atgatccgat gcttgatttc     780

agagtgttta aatatgacat tttctcactc tcgagtgtca ttaatatcat tattacagtg     840

gcactttaca ctggtatgtt ccttttaccg atttatctgc aaaacctggt cggctttacg     900

gcgcttcaat ccggtctgct tctgcttccg ggtgcgatcg ttatgctgat tatgtcaccg 960

atctcaggta ttttgttcga taaattcgga ccgagaccgc ttgcgattat cgggcttctc 1020

gtgacagtgg taacaacgta tcaatacacg caattgacaa ttgatacacc atatacacac 1080

atcatgctga tctattcaat tcgtgcattt ggtatgtctc tcttaatgat gccagtcatg 1140

acagccggga tgaaccagct tcctgcccgt ctgaacagcc acggaacggc gatgagtaac 1200

acgcttcgcc aaatcagcgg atcaatcgga acaagtttaa ttacgacgat ttatacaaac 1260

cggacgacat tccattattc acaaattgca gataaaacaa gcacggcgga cccgaacttc 1320

ctccatgcgt tccaaaatgc agtttcgaat ttaatggtga atatgaatgt gtcttatgat 1380

acggcgaaaa catatgtgta ctcacatatt tataaacacg catcactcga ttccaatgtc 1440

atggggataa atgatgcctt tatgtggcca accttattct gtgtagcagg tttgatttta 1500

agtatcttcc tgcgtgacgt cagaaaagat aaattgcgta agaagaaaaa agaggagctt 1560

tcattacttc ctgctccaaa agaagctaag gaatct 1596

<210> 5
<211> 639
<212> DNA
<213> Bacillus subtilis

<400> 5
atgtcattgg atgatctcca agcggcaaca aagattcaaa aaaggtattt aaccgccttg 60

gaggaaggaa actatgacat tattccgggg aaagttttat gttcgcattc atcaagcaat 120

atgcagaagc cgttggccaa tgatgcggat cagctgtttg aggagcataa aaaagatatc 180

cctaatacgt atcatgatga tgtgtctgaa aagatctccg gcatgaatct tcaaaaggaa 240

atgccaaagc cagcatctaa ggcgctggag ctgctgccaa caatacttgt tattctcggc 300

gtgattgtcg tgattgccat tgtgtacgcg atcattcaat ttggcaatca caaaaacagt 360

gatgatcaca atgcgggatc agaaaaagca attacgcaaa gtgagagcaa gtacgaaatc 420

cctaaagatt caacgctgaa agagaatcaa aataacagct ctgaaaaaga gacagacact 480

aaaaaagaaa cgaaagaaaa cgaagataag aaaaaagaaa atgacagtga gaagctggag 540

attaacgctg ctggcactga aggatcgtta acgacttatg aagtgtccgg cgctgataaa 600

atcgacctgg aactgaaggc atcagacagc tcctggatt 639

<210> 6
<211> 483
<212> DNA
<213> Bacillus subtilis

<400> 6
atggaaaact ttatcggaag ccacatgatt tatacgtatg aaaacggatg ggaatacgag 60

atttatatta aaaacgacca tacaattgat tatagaattc atagcggaat ggttgccgga    120

cgctgggttc gagatcagga agtgaatatt gtcaaactga cagaaggcgt atataaagtg    180

tcttggacag agccgactgg cacggatgtt tcattaaact ttatgccaaa tgaaaaacgc    240

atgcatggca ttattttctt cccgaaatgg gtgcatgaac atcctgaaat tacggtttgc    300

taccaaaatg accacattga tttgatgaaa gaatcccgcg aaaaatatga aacgtatcca    360

aaatacgttg tacctgaatt tgcggaaatt acatttctga aaaatgaagg agtcgacaac    420

gaagaagtga tttcgaaggc tccttatgag ggaatgacag acgatattcg cgcgggaaga    480

tta                                                                  483

<210>  7
<211>  21
<212>  DNA
<213>  artificial

<220>
<223>  primer

<400>  7
ggccgattca ttaatgcagg g                                               21

<210>  8
<211>  21
<212>  DNA
<213>  artificial

<220>
<223>  primer

<400>  8
cgatattcac ggtttaccca c                                               21

<210>  9
<211>  246
<212>  PRT
<213>  Aequora victoria

<400>  9

Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Thr Phe
        35                  40                  45

Ile Cys Thr Thr Gly Lys Leu Pro Val Pro Trp Pro Thr Leu Val Thr
    50                  55                  60

Thr Phe Ser Tyr Gly Val Gln Cys Phe Ser Arg Tyr Pro Asp His Met
65                  70                  75                  80

Lys Arg His Asp Phe Phe Lys Ser Ala Met Pro Glu Gly Tyr Val Gln
                85                  90                  95

Glu Arg Thr Ile Phe Phe Lys Asp Asp Gly Phe Thr Asn Tyr Lys Thr
            100                 105                 110

Arg Ala Glu Val Lys Phe Glu Gly Asp Thr Leu Val Asn Arg Ile Glu
        115                 120                 125

Leu Lys Gly Ile Asp Phe Lys Asp Asp Gly Asn Ile Leu Gly His Lys
    130                 135                 140

Leu Glu Tyr Asn Tyr Asn Glu His Leu Val Tyr Ile Met Ala Asp Lys
145                 150                 155                 160

Gln Lys Asn Gly Thr Lys Ala Phe Thr Ile Phe Gln Val His His Asn
                165                 170                 175

Ile Glu Asp Gly Gly Val Gln Leu Ala Asp His Tyr Gln Gln Asn Thr
            180                 185                 190

Pro Ile Gly Asp Gly Pro Val Leu Leu Pro Asp Asn His Tyr Leu His
        195                 200                 205

Thr Gln Ser Ala Leu Ser Lys Asp Pro Asn Glu Lys Arg Asp His Met
    210                 215                 220

Val Phe Leu Glu Phe Thr Phe Val Thr Ala Ala Gly Ile Thr His Gly
225                 230                 235                 240

Met Asp Glu Leu Tyr Lys
                245

<210>  10
<211>  717
<212>  DNA
<213>  Aequora victoria

<400>  10
atgagtaaag gagaagaact tttcactgga gttgtcccaa ttcttgttga attagatggt      60

gatgttaatg ggcacaaatt ttctgtcagt ggagagggtg aaggtgatgc aacatacgga     120

aaacttaccc ttaaatttat ttgcactact ggaaaactac ctgttccatg gccaacactt 180

gtcactactt tctcttatgg tgttcaatgc ttttcaagat acccagatca tatgaaacgg 240

catgactttt tcaagagtgc catgcccgaa ggttatgtac aggaaagaac tatatttttc 300

aaagatgacg ggaactacaa gacacgtgct gaagtcaagt ttgaaggtga tacccttgtt 360

aatagaatcg agttaaaagg tattgatttt aaagatgatg gaaacattct tggacacaaa 420

ttggaataca actataacga gcacttggtg tacatcatgg cagacaaaca aaagaatggt 480

accaaagcta tctttcaagt tcaccacaac attgaagatg gaggcgttca actagcagac 540

cattatcaac aaaatactcc aattggcgat ggccctgtcc ttttaccaga caaccattac 600

ctgcacacac aatctgccct ttcgaaagat cccaacgaaa agagagacca catggtcttt 660

cttgagtttg taacagctgc tgggattaca catggcatgg atgaactata caaataa 717